(19) Europäisches Patentamt
European Patent Office
Office européen des brevets

(11) EP 1 085 884 B1

(12) EUROPÄISCHE PATENTSCHRIFT

(45) Veröffentlichungstag und Bekanntmachung des Hinweises auf die Patenterteilung:
**09.03.2005 Patentblatt 2005/10**

(21) Anmeldenummer: **99927815.3**

(22) Anmeldetag: **31.05.1999**

(51) Int Cl.[7]: **A61K 33/04**, A61K 31/495, A61K 31/40
// A61K33:04, A61K31:40,(A61K31/495, A61P35:00), A61K31:40,(A61K31/495, A61P35:00, A61K31:513, 31:407), (A61K33/04, 31:407), (A61K33/04, 31:513, 31:407), (A61K33/04, 31:407), (A61K33/04, 31:513)

(86) Internationale Anmeldenummer:
**PCT/EP1999/003771**

(87) Internationale Veröffentlichungsnummer:
**WO 1999/064018 (16.12.1999 Gazette 1999/50)**

(54) **KOMBINATION VON SELEN-HALTIGEN VERBINDUNGEN MIT GEMCITABIN ODER MITOMYCIN C**

COMBINATION OF COMPOUNDS CONTAINING SELENIUM AND GEMCITABINE OR MITOMYCIN C

COMBINAISON DE COMPOSES CONTENANT DU SELENIUM AVEC DE LA GEMCITABINE OU DE LA MITOMYCINE C

(84) Benannte Vertragsstaaten:
**AT BE CH DE DK ES FI FR GB GR IE IT LI LU NL PT SE**

(30) Priorität: **09.06.1998 DE 19825746**

(43) Veröffentlichungstag der Anmeldung:
**28.03.2001 Patentblatt 2001/13**

(73) Patentinhaber: **Biosyn Arzneimittel GmbH
70734 Fellbach (DE)**

(72) Erfinder:
- **STIEFEL, Thomas
D-70184 Stuttgart (DE)**
- **RÖHRER, Helmut
D-79206 Breisach (DE)**

(74) Vertreter: **Grünecker, Kinkeldey,
Stockmair & Schwanhäusser Anwaltssozietät
Maximilianstrasse 58
80538 München (DE)**

(56) Entgegenhaltungen:
**EP-A- 0 750 911** **DE-A- 4 024 885**

- **KRAMER K.: "[Antioxidants in oncology]. ANTIOXIDANZIEN IN DER ONKOLOGIE." DEUTSCHE ZEITSCHRIFT FUR ONKOLOGIE, (1994) 26/3 (76-83). , XP002116032**
- **GUSTAFSON D L ET AL: "Inhibition of mitomycin C's aerobic toxicity by the seleno -organic antioxidant PZ-51." CANCER CHEMOTHERAPY AND PHARMACOLOGY, (1991) 28 (3) 228-30. , XP002116033**
- **STRAMA H. ET AL: "[An interesting case from oncological practice]. DER INTERESSANTE FALL." ZEITSCHRIFT FUR ONKOLOGIE, (1997) 29/1 (24-25). , XP002116034**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**(Forts. nächste Seite)**

- KIREMIDJIAN-SCHUMACHER L. ET AL: "Regulation of cellular immune responses by selenium." BIOLOGICAL TRACE ELEMENT RESEARCH, (1992) 33/1 (23-35). , XP002116035
- DOROSHOW J H ET AL: "Role of the glutathione-glutathione peroxidase cycle in the cytotoxicity of the anticancer quinones." PHARMACOLOGY AND THERAPEUTICS, (1990) 47 (3) 359-70. REF: 146 , XP002116036

## Beschreibung

Die vorliegende Erfindung bezieht sich auf die Verwendung von Selen und/oder einem Derivat davon in Kombination mit dem Zytostatikum Gemcitabin oder dem Zytostatikum Mitomycin C.

Das chemische Element Selen ist ein für Menschen und Tiere essentielles Spurenelement, das vor allem oxidative Prozesse sowie den Thyroxinmetabolismus beeinflußt. Beim Menschen konnte nachgewiesen werden, daß das Enzym Glutathionperoxidase und das im Plasma auftretende Selenoprotein P jeweils Selen in Form der Aminosäure Selenocystein enthalten. Die Selen-haltige Glutathionperoxidase ist Bestandteil des antioxidativen Schutzsystems der Säugetierzelle. In Gegenwart ausreichender Mengen an Substrat, d.h. reduziertem Glutathion, konvertiert die Glutathionperoxidase eine Vielzahl verschiedener Hydroperoxide zu entsprechenden Alkoholen. Es konnte gezeigt werden, daß die Integrität zellulärer und subzellulärer Membranen entscheidend von der Intaktheit des Glutathionperoxidase-Systems abhängt. Selen als Bestandteil der Glutathionperoxidase kann die Lipidperoxidationsrate und daraus resultierende Membranschäden senken.

In Tieren wurde kürzlich die Typ-I-Iodthyronin-5'-Deiodase als Selen-haltiges Enzym charakterisiert. Die Iodthyronin-Deiodase wandelt auch beim Menschen in der Schilddrüse, Leber und Niere Thyroxin ($T_4$) in Triiodthyronin ($T_3$), das aktive Schilddrüsenhormon, um. Bei Selenmangel, z.B. bei Phenylketonurie und zystischer Fibrose, konnten erhöhte $T_4$-Werte bei gleichzeitig reduziertem $T_3$-Spiegel nachgewiesen werden. Durch die Gabe von Natriumselenit ($Na_2SeO_3$) normalisiert sich der Schilddrüsenstoffwechsel wieder.

Als weiteres selenabhängiges Enzym wurde kürzlich eine humane Thioredoxinreduktase aus Lungenzellen beschrieben, die Selen als Ko-Faktor enthält (Tamura und Stadtman, 1996, Biochemistry, Proc. Natl. Acad. Sci., 93: 1006-1011). Das Enzym konnte bislang aus T-Zellen, Lungengewebe und Plazenta isoliert werden (Gladyshev et al., 1996, Biochemistry, Proc. Natl. Acad. Sci., 93: 6146-6151). Das selenabhängige Enzym Thioredoxinreduktase reduziert Thioredoxin. Thioredoxin wird bei einer Reihe von Tumoren überexprimiert, und einige experimentelle Studien haben gezeigt, daß Thioredoxin zum Wachstum und zur malignen Transformation einiger humaner Krebszellen beiträgt. Das Enzym Thioredoxinreduktase spielt deshalb eine Rolle in der Regulation des Wachstums von Normal- und Krebszellen.

Die pathophysiologische Relevanz der Selen-abhängigen Reaktionen ist durch Beobachtung von Selenmangelerscheinungen bei Menschen und Tieren belegt. Ein Mangel an diesem Spurenelement verstärkt oxidativ oder chemisch induzierte Leberschäden sowie die Toxizität von Schwermetallen wie Quecksilber und Cadmium.

Als Selenmangelerscheinungen werden beim Menschen die Keshan-Krankheit, eine endemisch auftretende Kardiomyopathie, und die sogenannte Kaschin-Beck-Krankheit beschrieben, eine ebenfalls endemisch auftretende Osteoathropathie mit starken Verformungen der Gelenke. Klinisch manifestierter Selenmangel wurde auch als Folge von langandauernder parenteraler Ernährung und von bilanzierten Diäten beobachtet. Dabei traten vor allem Kardiomyopathien und Myopathien der Skelettmuskulatur sowie eine Verschiebung des $T_3/T_4$-Verhältnisses auf.

Epidemiolische Untersuchungen deuten auf eine inverse Korrelation zwischen Blut-Selen-Spiegel und der Inzidenz von Herz-Kreislauf-Erkrankungen (Kardiomyopathien, Arteriosklerose, Myocardinfarkt) sowie von Tumorerkrankungen, besonders des Verdauungstraktes, der Brust und der Leber hin. Erniedrigte Plasma-Selenspiegel können bei Patienten mit Niereninsuffizienz sowie bei gastrointestinalen Erkrankungen vorliegen. Ein Selenmangel kann durch einen erniedrigten Vollblut- oder Plasma-Selen-Spiegel und durch erniedrigte Glutathionperoxidase-Aktivität in Vollblut, Plasma oder Thrombozyten nachgewiesen werden.

Selensubstitution bei Mangelerscheinungen aktiviert Reaktionen der Immunabwehr, insbesondere die unspezifischen, zellgebundenen und humoralen Reaktionen. Die Selenhaltige Glutathionperoxidase beeinflußt den Leukotrien-, Thromboxan- und Prostacyclin-Stoffwechsel. Im folgenden sind die immunmodulatorischen Effekte von Selenhaltigen Verbindungen aufgeführt:

- Stimulation der Lymphozyten-Proliferation
- Aktivierung von zytotoxischen T-Zellen und NK-Zellen
- Erhöhung der Interleukin-2-Rezeptorexpression
- selektive Senkung der T-Suppressorzellzahl
- Erhöhung der Interferon-γ-Synthese
- allgemeine Senkung der Infektionshäufigkeit

Selen in Form von Selenit ($SeO_3^{2-}$) wird nicht direkt in Proteine eingebaut. Im Blut wird Selenit zuerst hauptsächlich von den Erythrozyten aufgenommen und enzymatisch zu Selenwasserstoff reduziert. Selenwasserstoff dient als zentraler Selen-Pool für die Ausscheidung und für den gezielten Einbau in Selenoproteine. In dieser reduzierten Form wird Selen an Plasmaproteine gebunden, die in die Leber und andere Organe wandern. Der von der Leber ausgehende plasmatische Sekundärtransport in die Glutathionperoxidase-synthetisierenden Zielgewebe geschieht wahrscheinlich in Form eines Selenocystein-haltigen P-Selenoproteins. Der weitere metabolische Verlauf der Selenoprotein-Biosynthese ist bisher nur aus prokaryontischen Modellorganismen bekannt. In diesen wird Selenocystein im Verlauf der Translation spezifisch in die Peptidkette der Glutathionperoxidase eingebaut.

Überschüssiger Selenwasserstoff wird beim Menschen über Methylselenol und Dimethylselenid zum Trimethylselenonium-Ion, dem Hauptausscheidungsprodukt, metabolisiert. Selenit wird nach oraler Applikation vorwiegend aus dem Dünndarm absorbiert. Die intestinale Absorption von Natriumselenit ist nicht homöostatisch reguliert. Sie beträgt in Abhängigkeit von der Konzentration und von Begleitsubstanzen zwischen 44% und 89%, gelegentlich über 90%. Die Aminosäure Cystein fördert die Natriumselenit-Absorption.

Organische Selenverbindungen müssen ebenfalls zuerst in Selenwasserstoff umgewandelt werden, bevor sie für die Synthese von Selenoproteinen zur Verfügung stehen. Das in der Nahrung hauptsächlich enthaltene Selenomethionin kann auch anstelle von Methionin bei der Proteinbiosynthese unspezifisch in nicht selenhaltige Proteine statistisch eingebaut werden.

Die Gesamtmenge an Selen im menschlichen Körper liegt bei einem ausgeglichenen Selen-Haushalt zwischen 4 mg und 20 mg. Die Ausscheidung von Selen erfolgt beim Menschen je nach applizierter Dosis über den Urin, die Fäzes und über die Lunge. In erster Linie wird Selen in Form der oben erwähnten Trimethylselenonium-Ionen renal ausgeschieden.

Beim Menschen wurden akute Selenintoxikationen bisher selten beschrieben. Als Anzeichen einer akuten Überdosierung gelten knoblauchartiger Atemgeruch, Müdigkeit, Übelkeit, Diarrhö und abdominelle Schmerzen. Aus Beobachtungen zur chronischen Toxizität von Selen beim Menschen wurde eine maximale sichere tägliche Aufnahme von Selen von 820 µg abgeleitet, wahrend eine Dosierung von bis zu 550 µg pro Tag auch bei empfindlichen Personen als unbedenklich angesehen wird. Als klinische Anzeichen der endemisch auftretenden Selenose wurden bei einer Studie in China nach täglicher Zufuhr von 3200-6700 µg Selen Haarausfall, Brüchigkeit der Fingernägel, Hautveränderungen und Störungen des Nervensystems beobachtet. Bei verschiedenen Spezies wurde als Symptom der Selenose eine Einschrankung der Reproduktionsfähigkeit aufgrund verringerter Motilität der Spermatozoen beschrieben.

In einer Dosis-Eskalationsstudie wurden bei Tumorpatienten zwischen 10 und 50 mg Selen in Form von Natriumselenit-Pentahydrat infundiert. Innerhalb von 30 Minuten stieg der Plasmaselenspiegel nach Gabe von 10 mg Selen als Natriumselenit von 200 µg/l auf 1200 µg/l. Nach 8 und 16 Stunden war das Plasmaselen auf 770 bzw. 430 µg/l abgesunken. Nach 24 Stunden hatte der Plasmaselenspiegel wieder seinen Ausgangswert erreicht. Die gastrointestinale Toxizität trat ab etwa 20 mg Selen als Natriumselenit auf und war nach Absetzen des Präparates reversibel (Röhrer H., 1989, Erfahrungsheilkunde 38: 10a, 761).

Als Gegenmaßnahmen im Falle einer Vergiftung kommen Magenspülung, erzwungene Diurese oder hochdosierte Vitamin C-Gaben in Frage. Bei extremer Überdosierung (1000-10000-fach) kann versucht werden, das Selenit durch Dialyse zu eliminieren.

Das Spurenelement Selen wird von Menschen vorwiegend durch den Verzehr von Eigelb Fisch und Fleisch, besonders vom Huhn und Schwein, sowie Innereien aufgenommen. Die minimale notwendige Selenzufuhr des Menschen hängt ab von der chemischen Form des aufgenommenen Elementes und von der Zusammensetzung der Diät, in der es vorliegt. In China wurde experimentell eine Menge von 15-20 µg Selen pro Tag als ausreichend ermittelt, um vor endemischen Selenmangelkrankheiten zu schützen. Der National Research Council (NRC) der USA empfiehlt für Männer eine tägliche Zufuhr von 70 µg Selen, für Frauen 55 µg Selen. Der NRC stufte früher (bis 1989) Tagesmengen von 50-200 µg Selen als angemessen und unbedenklich ein. Die deutsche Gesellschaft für Ernährung empfiehlt 20-100 µg Selen täglich.

Die tägliche durchschnittliche Selenzufuhr, zu 2/3 gedeckt durch die Zufuhr von tierischem Eiweiß, liegt in den alten Bundesländern Deutschlands bei 38 µg für Frauen und 47 µg für Männer. Im Gebiet der neuen Bundesländer wurden hingegen Werte von 20-25 µg Selen ermittelt. Diese Zahlen belegen, daß die nutritive Selenzufuhr in Deutschland nicht immer gedeckt ist. Das Risiko einer unzureichenden Versorgung mit Selen besteht besonders in Situationen mit erhöhtem Bedarf (z.B. Schwangerschaft und Stillzeit), bei Personen unter Schwermetall- und Oxidanzienbelastung, bei Patienten mit gastrointestinalen Komplikationen (z.B. chronisch entzündliche Darmerkrankungen) und bei parenteral oder mit besonderen Diäten (z.B. bei Phenylketonurie) ernährten Personen.

Epidemiologische Studien haben gezeigt, däß geringe Selenaufnahme und entsprechend geringe Selen-Blutplasmakonzentration mit einem erhöhten Auftreten verschiedener Krebsarten beim Menschen in Zusammenhang stehen (Glattre et al., 1989, Int. J. Epedemiol., 18:45-49; Knekt et al., 1990; J. Natl. Cancer Inst., 82:864-868; Burney et al., 1997, J. Clin. Nutr., 49:895-900). Es wurde ebenfalls gezeigt, daß Selen in hohen Dosen (10-100 µM) das Wachstum verschiedener Tumorzellen, z.B. bei menschlichen Brustkrebszellen, Eierstockkrebszellen und Darmkrebszellen, in vitro deutlich inhibieren kann (Yan et al., 1991; Biol. Trace Elem. Res., 30:145-162; Chen et al., 1995, FASEB J., 9(3): A159; Nano et al., 1989, Biol. Trace Elem. Res. 20: 31-43; Stewart et al., 1997, Cancer Lett., 117:35-40). Im Gegensatz dazu berichten mehrere Wissenschaftler von einem wachstumsstimulierenden Effekt geringer Mengen an Natriumselenit (0,001-1 µM) auf verschiedene Tumorzellen, die unter serumfreien Bedingungen inkubiert wurden (Nano et al., 1989, Biol. Trace Elem, Res., 20:31-43; Golczewski und Frenkel, 1989, Biol Trace Elem. Res. 20:115-126). Des weiteren wurde beobachtet, daß organische Selenverbindungen eine praventive Wirkung in bezug auf die Tumorentwicklung von Mammakarzinomen in Mäusen und Ratten ausüben (El-Bayoumy et al., 1995, J. Cell. Biochemistry, Anhang 22: 29-100). Der Mechanismus, durch den Selen die Tumorproliferation bzw. -regression beeinflußt, ist nicht näher bekannt. Es wird jedoch vermutet, daß die Induktion von DNA-Strangbrüchen und Apoptose, welche durch Selen und/oder Selenmetabolite, wie Selenodiglutathion und Hydrogenselenid sowie durch die Bildung von Selenoproteinen, wie Glutathionperoxidase und Thioredoxinreduktase eine wichtige Rolle dabei spielen (Thompson et al., 1994, Carcinogenesis 15:183-186; Wu et al., 1995, Carcinogenesis 16: 1579-1584; Lu et al., 1994, Biochem. Pharmacol., 47: 1531-1535; Milner, 1985, Fed. Proc., 44: 2568-2572 ; Schrauzer, 1992, Biol. Trace Elem. Res., 33:51-62; Gallegos et al., 1997, Cancer Res., 75:4965-4979). So könnte z.B. eine Erhöhung der Thioredoxinreduktase-Aktivität durch Selen die zelluläre Thioredoxinkonzentation senken und daher die Regulation des Wachstums von Krebszellen beeinflussen (Gallegos et al., 1997, Cancer Res., 75: 4965-4979).

In Kombinationsexperimenten wurde beobachtet, daß die Verabreichung geringer Mengen an Selen bzw. Selen-haltigen Verbindungen zusammen mit Zytostatika keine Senkung des antitumoralen Effektes bewirkt, jedoch die durch Zytostatika bedingten Nebenwirkungen, wie Nephrotoxizität oder Kardiotoxizität erheblich zu reduzieren vermag.

Während für einige Krebsarten bereits recht wirksame Therapieverfahren entwickelt werden konnten - die Mortalitätsrate nach Darmkrebserkrankung konnte z.B. zwischen 1992 bis 1993 um ungefähr 17% gesenkt werden - steht für die überwiegende Mehrheit von Krebsarten bisher noch gar keine oder eine nur sehr unzureichende Therapie zur Verfügung.

Neben der operativen Entfernung des Tumors und der Strahlentherapie gilt die Chemotherapie als das bisher wirksamste Therapieverfahren. Chemotherapeutische Medikamente lassen sich im wesentlichen in die folgenden vier Gruppen einteilen: Antimetabolite, Topoisomerase-Inhibitoren, alkylierende Mittel und Pflanzenalkaloide, wobei die drei erstgenannten Gruppen eine korrekte Replikation der Erbsubstanz verhindern und die letzte Gruppe Mitose-inhibierend wirkt. Bei der Behandlung vor allem solider Tumore reicht die Wirkung der Zytostatika meistens nicht aus, um die Tumore kurativ zu behandeln.

Es ist daher Aufgabe der vorliegenden Erfindung, eine Möglichkeit anzugeben, bei antitumoralen Wirkstoffen eine Wirkungssteigerung zu erzielen sowie diese Wirkstoffe in geeigneter Darreichungsform bereitzustellen.

Diese Aufgabe wird durch die Verwendung von Selen und/oder mindestens einer Selenverbindung zur Wirkungssteigerung des Zytostatikums Gemcitabin oder des Zytostatikums Mitomycin C gelöst.

Weiterhin wird diese Aufgabe gelöst durch die Bereitstellung eines Kits, umfassend

a) Selen und/oder mindestens eine Selenverbindung und
b) das Zytostatikum Gemcitabin oder das Zytostatikum Mitomycin C,

als Kombinationspräparat zur gleichzeitigen, getrennten oder zeitlich abgestuften Anwendung in der zytostatischen Therapie, dadurch gekennzeichnet, dass Selen und/oder die Selenverbindung in einer Konzentration von 0,1 mg/kg Körpergewicht bis 1,25 mg/kg Körpergewicht, und das Zytostatikum in einer Konzentration von 2 $mg/m^2$ Körperoberfläche bis 240 $g/m^2$ Körperoberfläche verwendet wird.

Weiterhin wird diese Aufgabe gelöst durch die Verwendung von Selen und/oder mindestens einer selenhaltigen Verbindung in Kombination mit dem Zytostatikum Gemcitabin oder mit dem Zytostatikum Mitomycin C zur Herstellung eines Medikaments zur Behandlung von Krebs, dadurch gekennzeichnet, dass Selen und/oder die Selenverbindung in einer Konzentration von 0.1 mg/kg Körpergewicht bis 1,25 mg/kg Körpergewicht, und das Zytostatikum in einer Konzentration von 2 $mg/m^2$ Körperoberfläche bis 240 $g/m^2$ Körperoberfläche verwendet wird. Die vorliegende Erfindung bezieht sich auf die Verwendung von Selen und/oder mindestens einer Selenverbindung zur Wirkungssteigerung zweier Zytostatika, d.h. von Gemcitabin oder Mitomycin C, oder einer Mischung davon. Die im folgenden aufgeführten Beispiele demonstrieren, daß in vitro eine gleichzeitige Behandlung mit den oben genannten Komponenten überraschend zu einem deutlichen synergistischen, d.h. überadditiven antitumoralen Effekt führt.

Es werden organische und anorganische Selenverbindungen zur Kombination mit den oben genannten Zytostatika eingesetzt. In einer bevorzugten Ausführungsform wird eine organische Selenverbindung verwendet. Die Verwendung von organischen Selenverbindungen soll dabei der Senkung der Toxizität gegenüber anorganischen Selenverbindungen bei gleicher oder verbesserter antitumoraler Wirksamkeit dienen. Besonders bevorzugt sind die Selenaminosäuren Selenomethionin und Selenocystein sowie die Verbindung Phenylenbis(methylen)selenocyanat sowie Derivate davon (El-Bayonmi et al., 1995, J. Cell. Biochemistry, Anhang 22: 92-100). Die letztgenannte Verbindung inhibiert Thymidinkinase in menschlichen Mammakarzinomzellinien. Des weiteren wurde darüber berichtet, daß diese Verbindung eine Hemmung des Zellwachstums sowie die Induktion des Zelltods durch Apoptose auslösen kann.

Weiterhin wird ein Selenoxid als Selenverbindung bevorzugt, um eine Wirkungssteigerung eines Zytostatikums oder einer Mischung von Zytostatika zu erzielen. In einer besonders bevorzugten Ausführungsform ist die Selenverbindung ein Salz von $SeO_2$, z.B. das Salz $Na_2SeO_3$.

Ein bevorzugtes Nukleinsäuresynthese-hemmendes Zytostatikum ist Gemcitabin. Ein weiteres ebenfalls bevorzugtes Nukleinsäuresynthese-hemmendes Zytostatikum ist die Verbindung Mitomycin C. Im folgenden sind die Strukturformeln dieser beiden Verbindungen dargestellt:

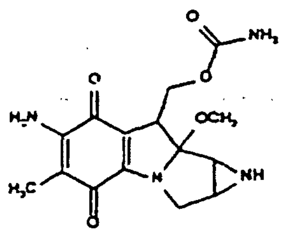
Mitomycin C

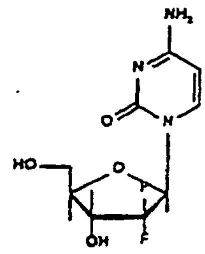
Gemcitabin

Mitomycin C gehört zur Gruppe der alkylierenden Mittel. Nach Reduktion der Chinon-Einheit wird Methanol freigesetzt, was die Öffnung des Aziridinringes zur Bildung eines alkylierenden Metaboliten erleichtert. Ein weiteres alkylierendes Molekül wird durch chemische oder enzymatische Abtrennung der Carbamatseitenketten gebildet. Darüber hinaus ist die Reduktion der Chinon-Einheit an die Bildung reaktiver Sauerstoffmoleküle gebunden, welche ebenfalls einen alkylierenden Effekt haben. Die antitumorale Wirkung von Mitomycin ist hauptsächlich auf die Alkylierung von DNA zurückzuführen.

Gemcitabin ist ein Pyrimidinantimetabolit. Nach Aufnahme in die Zelle wird es zu 2',2'-Difluordeoxycytidintriphosphat metabolisiert. Die Inkorporation von Gemcitabin in die DNA unterbricht die DNA-Strang-Synthese, so daß folglich keine Zellteilung mehr möglich ist.

Gemcitabin und Mitomycin C haben unterschiedliche Wirkweisen, doch interagieren beide Verbindungen direkt mit zellulärer DNA und führen zu Fehlern bzw. dem Abbruch der DNA-Replikation.

Zur Herstellung eines Medikaments zur Behandlung von Krebs wird Selen und/oder mindestens eine Selenverbindung in einer Konzentration von 0,1 mg/kg Körpergewicht bis 1,25 mg/kg Körpergewicht, und das Zytostatikum in einer Konzentration von 2 $mg/m^2$ Körperoberfläche bis 240 $g/m^2$ Körperoberfläche verwendet. Dabei liegt die bevorzugte Konzentration von Selen bzw. einer Selenverbindung in einem Bereich von 0,1 mg/kg Körpergewicht bis 0,3 mg/kg Körpergewicht, und die des Zytostatikums in einem Bereich von 20 $mg/m^2$ Körperoberfläche bis 1000 $mg/m^2$ Körperoberfläche.

Der Einsatz der genannten Kombination in der zytostatischen Therapie ist besonders bevorzugt.

Des weiteren stellt die Erfindung einen Kit zur Verfügung, welcher Selen und/oder mindestens eine Selenverbindung und ein Zytostatikum oder eine Mischung von Zytostatika als Kombinationspräparat zur gleichzeitigen, getrennten oder zeitlich abgestuften Anwendung in der zytostatischen Therapie umfaßt. Dabei ist bevorzugt, daß die im Kit enthaltene Selenverbindung eine organische Selenverbindung ist. Besonders bevorzugte organische Selenverbindungen sind die Selenaminosäuren Selenomethionin und Selenocystein sowie die Verbindung Phenylenbis(methylen)selencyanat. Darüber hinaus wird in einer weiteren Ausführungsform als Selenverbindung ein Selenoxid bevorzugt. Besonders bevorzugt ist dabei ein Salz von $SeO_2$, z.B. $Na_2SeO_3$.

Der erfindungsgemäße Kit enthält Selen und/oder mindestens eine Selenverbindung, die in einer Konzentration von 0,1 mg/kg Körpergewicht bis 1,25 mg/kg Körpergewicht zu verabreichen sind, und eines der oben beschriebenen Zytostatika, das in einer Konzentration von 2 $mg/m^2$ Körperoberfläche bis 240 mg/kg Körperoberfläche zu verabreichen ist. Dabei ist ein Konzentrationsbereich von 0,1 mg/kg Körpergewicht bis 0,3 mg/kg Körpergewicht für Selen bzw. eine Selenverbindung und ein Konzentrationsbereich von 20 mg/kg Körpergewicht bis 1000 $mg/m^2$ Körperoberfläche eines wie oben charakterisierten Zytostatikums bevorzugt.

Die Kombinationen aus Selenverbindung und Zytostatikum bzw. Zytostatika können in fester oder flüssiger Form verabreicht werden. Dabei kann die Applikation oral, rectal, nasal, topical (einschließlich buccal und sublingual), vaginal oder parenteral (einschließlich intramuskulär, subcutan und intravenös) oder durch Inhalation erfolgen. Sie können dabei zusammen mit herkömmlichen Adjuvantien, Trägerstoffen und/oder Verdünnungsmitteln angeboten werden.

Die festen Darreichungsformen umfassen Tabletten, Kapseln, Pulver, Pillen, Pastillen, Suppositorien und gekörnte Darreichungsformen. Sie können ebenfalls Zusatzstoffe, wie Geschmacksstoffe, Farbstoffe, Verdünnungsmittel, Weichmacher, Bindemittel, Konservierungsmittel, Sprengmittel und/oder umschließende Materialien miteinschließen.

Flüssige Darreichungsformen umfassen Lösungen, Suspensionen und Emulsionen. Diese könne ebenfalls zusammen mit den obengenannten Zusatzstoffen angeboten werden.

Die folgenden Figuren und Beispiele erläutern die vorliegende Erfindung.

Fig. 1: Effekt von Natriumselenit (3 µM und 30 µM) auf die Koloniebildung von pankreatischen Tumorxenotransplantaten (PAXF 546 und 736) in dem klonogenen in vitro-Test in verschiedenen Experimenten. T/C = Anzahl an Tumorkelonien in Selen-behandelten Proben geteilt durch die Anzahl an Kolonien in den unbehandelten Proben in %.

Fig. 2: Effekt von Selen oder Mitomycin C alleine oder zusammen auf das in vitro- Wachstum des pankreatischen Tumorxenotransplantates PAXF 546 (klonogener Test, kontinuierliche Wirkstoffexposition) A - C: Dosis/Wirkungs-Kurven von Mitomycin C und Mytomycin C in Kombination mit 3 µM Selen (A) oder 30 µM Selen (B, C). Der Effekt von Selen alleine ist durch gestrichelte Linien angedeutet.

Fig. 3: Vergleich der experimentell beobachteten und erwarteten T/C-Werte von Selen/Mitomycin C-Kombinationen. Erwartete T/C-Werte für einen additiven Wirkstoffeffekt sind als offene Balken dargestellt, experimentell beobachtete T/C-Werte als ausgefüllte Balken; * bezeichnete einen signifikanten Unterschied ($p < 0{,}01$) zwischen $T/C_{erwartet}$ und $T/C_{beobachtet}$ und daher einen Wirkstoffsynergismus. A-C: Kombination von Mitomycin C mit 3 µM Selen (A) oder 30 µM Selen (B, C).

Fig. 4: Effekt von Selen und Gemcitabin alleine oder in Kombination auf das in vitro- Wachstum des pankreatischen Tumorxenotransplantates PAXF 546 (klonogener Test, kontinuierliche Wirkstoffexposition). A-C: Dosis/ Wirkungs-Kurve von Gemcitabin alleine und Gemcitabin in Kombination mit 3 µM Selen (A) oder 30 µM Selen (B, C). Der Effekt von Selen alleine wird durch gestrichelte Linien angedeutet.

Fig. 5: Vergleich der experimentell beobachteten und erwarteten T/C-Werte von Selen/Gemcitabin-Kombinationen. Erwartete T/C-Werte für den additiven Wirkstoffeffekt sind als offene Balken dargestellt, experimentell beobachtete T/C-Werte als ausgefüllte Ballken; * weist auf einen signifikanten Unterschied ($p < 0{,}01$) zwischen $T/C_{erwartet}$ und $T/C_{beobachtet}$ und daher Wirkstoffsynergismus hin. A/C: Kombination von Gemcitabin mit 3 µM Selen (A) oder 30 µM Selen (B, C).

Beispiele:

## 1. Material und Methoden

Natriumselenit (Selenase®) wurde von der G. N. Pharm GmbH, Fellbach, Deutschland zur Verfügung gestellt. Die 0.9 %-ige NaCl-Lösung enthielt 50 µg/ml (0.63 mM) an Selen als $Na_2SeO_3 \times 5H_2O$. Gemcitabin und Mitomycin C wurden von einer Apotheke bezogen und als klinische Formulierungen benutzt.

### 1.1 Koloniebildungstest

Herstellung einer Einzelzellsuspension von menschlichen Xenotransplantaten

Solide menschliche Tumorxenotransplantate, die subkutan in seriellen Passagen in Thymus-aplastischen Nacktmäusen (NMRI nu/nu-Stamm: aus eigener Zucht) wuchsen, wurden unter sterilen Bedingungen entfernt, die Zellen mechanisch vereinzelt und anschließend mit einem Enzymgemisch, bestehend aus Kollagenase (1,2-1,8 E/ml, Worthington), DNAse (375 E/ml, Boehringer Mannheim) und Hyaluronidase (29 E/ml, Boehringer Mannheim) in RPMI 1640 bei 37°C für 30 Minuten inkubiert. Die Zellmischung wurde durch Siebe mit 200 µm und 50 µm Porengröße passiert und danach zweimal mit PBS gewaschen. Der Prozentsatz an lebenden Zellen wurde in einer Neubauer-Zellkammer unter Verwendung von Trypanblau-Ausschluß bestimmt.

### 1.2 Kultivierungsverfahren

Der klonogene Test wurde gemäß dem von Hamburger und Salmon beschriebenen modifizierten 2-Lagen Soft-Agar-Test durchgeführt (Hamburger und Salmon, Science, 197: 461-463, 1977). Die untere Schicht bestand aus 0,2 ml Iscoves's Modified Dulbecco's Medium mit 20% fötalem Kälberserum und 0,75% Agar. $8 \times 10^3$ bis $1{,}6 \times 10^4$ Zellen wurden zu 0,2 ml desselben Mediums zusammen mit 0,4% Agar zugesetzt und in bereits beschichtete 24-multiwell-Platten gegeben. Teststubstanzen wurden durch fortgesetzte Exposition (drug overlay) in 0,2 ml Medium zugesetzt. Jede Schale schloß 6 Kontrollvertiefungen ein, welche die mit Trägersubstanz und Testsubstanz behandelten Gruppen in 6 Konzentrationen in 3-facher Ausführung enthielten.

Die Kulturen wurden bei 37° und 7% $CO_2$ in einer humidifizierten Atmosphäre für 5 bis 15 Tage, abhängig von der Verdopplungszeit der Tumorstammzellen, inkubiert und das Koloniewachstum wurde unter einem Inversmikroskop beobachtet. In dieser Zeitspanne führte das in vitro-Tumorwachstum zu der Bildung von Kolonien mit einem Durchmesser von ≥ 50 µm. Wenn maximale Koloniebildung erreicht war, wurden Zählungen mit einem automatischen Bildanalysesystem (OMNICOM Fas IV, Biosys GmbH) durchgeführt. 24 Stunden vor diesem Test wurden lebende Kolonien mit einer sterilen wäßrigen Lösung an 2-(4-Iodphenyl)-3-(4-nitrophenyl)-5-phenyltetrazoliumchlorid (1 mg/ml, 100 µl/Vertiefung) angefärbt. Der Effekt der Testsubstanzen wurde dargestellt als Prozent der überlebenden Zellen, erhalten durch Vergleich der durchschnittlichen Koloniezahlen der behandelten Platten mit der durchschnittlichen Koloniezahl der unbehandelten Kontrolle (Test-versus-Kontroll-Gruppenwert, T/C = $Koloniezahl_{behandelte\ Gruppe}$* 100/$Koloniezahl_{Kontrollgruppe}$).

Ein Test wurde als aussagekräftig erachtet, wenn die folgenden Kontrollkriterien erfüllt waren:

- durchschnitliche Koloniezahl der Kontrollgruppenplatten für 24-multiwells ≥20 Kolonien mit einem Koloniedurchmesser von ≥ 50 µm.

- Kolonieüberlebenszahl in Vertiefungen, die mit der Positivreferenzverbindung 5-Fluoruracil (bei einer toxischen Dosis von 1000 µg/ml) behandelt wurden < 30% der Kontrollen.

- Koeffizient der Variation in der Kontrollgruppe ≤ 50%.
  $IC_{50}$ und $IC_{70}$-Werte wurden durch Auftragen der Verbindungskonzentration gegen Zellüberlebensrate bestimmt. Durchschnittliche $IC_{50}$ und $IC_{70}$-Werte wurden gemäß der folgenden Formel berechnet:

$$\text{Durchschnittlicher } IC_{50} \text{ und } IC_{70} = \frac{\sum_{x=1}^{n} \log (IC_{50,70})_x}{n}$$

mit x = spezifischem Tumorxenotransplantat und n = Gesamtzahl an untersuchten Tumorxenotransplantaten. Falls kein $IC_{50}$ oder $IC_{70}$-Wert mit dem untersuchten Dosisbereich bestimmt werden konnte, wurde die höchste bzw. niedrigste untersuchte Konzentration für die Berechnung herangezogen

1.3 Kombinationsstudien

Die Inhibition der Koloniebildung durch die Chemotherapeutika Gemcitabin und Mitomycin C alleine oder in Kombination mit Selen wurden in pankreatischen menschlichen Tumorxenotransplantaten PAXF 546 und 736, unter Verwendung des oben beschriebenen klonogenen Tests untersucht. Jedes Experiment umfaßt 6 Konzentrationen an Chemotherapeutika, 2 Konzentrationen an Selen (3 und 30 µM) und die Kombination aller Dosen mit Selen bei 3 und 30 µM. Alle Verbindungen und Kombinationen wurden dreifach untersucht. Konzentrationen der Chemotherapeutika wurden so gewählt, daß T/C-Werte von 0 bis 100% daraus resultierten. Jedes Experiment wurde zweimal durchgeführt. Die Wirkung einer Kombination von Verbindungen wurde durch Vergleich des experimentellen beobachteten T/C-Wertes einer Selen/Zytostatikum-Kombination ($T/C_{beobachtet}(A+B)$) mit dem erwarteten T/C-Wert für diese Kombination ($T/C_{erwartet}(A+B)$) bestimmt, berechnet durch Multiplizieren der Anzahl überlebender Kolonien, erhalten nach Behandlung der Zellen mit den jeweiligen Einzelsubstanzen (T/C (A) und T/C (B)) unter Verwendung der folgenden Gleichung (Multiplikationsverfahren z.B. beschrieben von Berenbaum, 1989, Pharmacol. Rev., 41:93-141):

$$T/C_{erwartet}(A+B) = T/C\ (A) \times T/C\ (B)/100$$

Für eine Null-interaktive Wirkstoffkombination (additive Wirkstoffeffekte) ist $T/C_{erwartet}$ (A+B) = $T/C_{beobachtet}$ (A+B). Eine Wirkstoffkombination ist synergistisch, wenn $T/C_{beobachtet}$ (A+B) statistisch signifikant > $T/C_{erwartet}$ (A+B) ist. Statistische Signifikanz wurde durch den T-Test bestimmt.

## 2. Ergebnisse

2.1. In vitro-Antitumoraktivität von Natriumselenit auf menschliche Tumorxenotransplantate

Die zytotoxische Aktivität von Natriumselenit wurde unter Verwendung des klonogenen Tests an den folgenden menschlichen Tumorxenotransplantaten untersucht:

Tabelle 1

| Untersuchte menschliche Tumorxenotransplantate | | |
|---|---|---|
| Tumor | | ursprüngliche Histologie |
| Lunge | LXFL 529 | nicht kleinzelliges Bronchialkarzinom |
| | LXFS 650 | kleinzelliges Bronchialkarzinom |
| Brust | MAXF 401NL | Adenokarzinom, Östrogenrezeptor negativ, Progesteronrezeptor negativ |
| | MAXF MCF7X | Adenokarzinom, Östrogenrezeptor negativ, Progesteronrezeptor negativ |
| Eierstock | OVXF 899 | Adenokarzinom |
| | OVXF 1353 | adenoides Karzinom |
| Pankreas | PAXF 546 | adenosquamöses Karzinom |
| | PAXF 736 | Adenokarzinom |
| Prostata | PC3MX | Adenokarzinom |
| | DU145X | Karzinom |
| Niere | RXF 393 | Hypernephrom |
| | RXF 944LX | Hypernephrom |

Es wurden Natriumselenitkonzentrationen zwischen 0,001 und 100 µM eingesetzt. T/C-Werte, die in den verschiedenen Tumorxenotransplantaten erhalten wurden, sind in Tabelle 2 gezeigt. $IC_{50}$ und $IC_{70}$-Werte sowie ein $IC_{70}$-Balkendiagramm, welches die Sensitivität der verschiedenen Tumore gegenüber Selenbehandlung zeigt, sind in Tabelle 3 angegeben.

Die Inhibition der Koloniebildung hängt von der Selendosis ab. Bis zu 1 µM konnte kein deutlicher zytotoxischer Effekt beobachtet werden. Bei 10 µM Selen wurden T/C-Werte zwischen 30% in 2 von 12 Xenotransplantaten (LXFL 529, PRXF DU145X) erhalten. Bei sehr hohen Selenkonzentrationen von 100 µM wurden T/C-Werte unter 20% in allen Xenotransplantaten erreicht, was auf einen unspezifisch zytotoxischen Effekt hinweist.

Tabelle 2

| In vitro-Effekt von Natriumselenit auf menschliche Tumorxenotransplantate | | | | | | | |
|---|---|---|---|---|---|---|---|
| TUMOR/PASSAGE Nr. | EXP. Nr. | Test/Kontrolle (%) bei Wirkstoffkonzentration [µM] | | | | | |
| | | .001 | .01 | .1 | 1. | 10. | 100. |
| LXFL | | | | | | | |
| 529/18 | X182AM | 90 - | 78 - | 72 - | 61 - | 9 +++ | 0 +++ |
| LXFS | | | | | | | |
| 650/9 | X199AM | 100 - | 100 - | 98 - | 84 - | 109 - | 16 ++ |
| MAXF | | | | | | | |
| 401/16 | X186AM | 67 s- | 81 s- | 53 - | 74 - | 66 - | 8 +++ |
| MCF7X/28 | X218AM | 93 - | 85 - | 90 - | 78 - | 97 - | 0 +++ |
| OVXF | | | | | | | |
| 899/33 | X185AM | 80 - | 79 - | 66 - | 70 - | 73 - | 1 +++ |
| 1353/17 | X217AM | 96 - | 91 - | 100 - | 90 - | 100 - | 0 +++ |
| PAXF | | | | | | | |
| 546/2 | *(2) | 82 - | 112 - | 100 - | 104 - | 88 - | 2 +++ |
| 736/17 | X184AM | 87 - | 77 - | 94 - | 88 - | 90 - | 4 +++ |
| PRXF | | | | | | | |
| PC3M/3 | X189/AM | 91 - | 91- | 89 - | 85 - | 38 + | 1 +++ |
| DU145X/15 | X229AM | 97 - | 96 - | 92 - | 91 - | 5 +++ | 0 +++ |
| RXF | | | | | | | |
| 393/9 | X194AM | 62 - | 45 - | 31 + | 31 + | 32 + | 10 +++ |
| 423/16 | X183AM | 99 - | 111 - | 120 - | 92 - | 110 - | 0 +++ |
| aktiv(++, +++)/gesamt | | 0/12 | 0/12 | 0/12 | 0/12 | 2/12 | 12/12 |

Tabelle 2 (fortgesetzt)

| In vitro-Effekt von Natriumselenit auf menschliche Tumorxenotransplantate | | | | | | | |
|---|---|---|---|---|---|---|---|
| TUMOR/PASSAGE Nr. | EXP. Nr. | Test/Kontrolle (%) bei Wirkstoffkonzentration [µM] | | | | | |
| | | .001 | .01 | .1 | 1. | 10. | 100. |
| nur Xenotransplantate | | 0% | 0% | 0% | 0% | 17% | 100% |

Tabellenlegende:
LXF Lungen A Adeno, L großzelliges, S kleinzelliges Krebsxenotransplantat;
MAXF Brustkrebsxenotransplantat: OVXF Eierstockkrebsxenotransplantat,
PAXF Pankreas, PRXF Prostatakrebsxenotransplantat; RXF Nierenkrebsxenotransplantat
- (T/C ≥ 50%); + (30% ≤ T/C < 50%); ++ (10% < T/C < 30%); +++ (T/C ≤ 10%),
s Ergebnis einer Platte

In dem $IC_{70}$-Balkendiagramm ($IC_{70}$-Darstellung Tabelle 3) sind Abweichungen von einzelnen $IC_{70}$-Werten von dem Durchschnittswert als Balken in logarithmischer Darstellung präsentiert. Balken nach links zeigen $IC_{70}$-Werte, die geringer als der Durchschnittswert sind, Balken nach rechts zeigen Werte, die höher als der Durchschnittswert sind. Die $IC_{70}$-Darstellung zeigt daher ein charkateristisches anti-proliferatives Profil der Verbindung.

Der durchschnittliche $IC_{50}$-Wert von Natriumselenit war 15,5 µM, der durchschnittliche $IC_{70}$-Wert 27 µM. Das entspricht einer Selenkonzentration von 1,2 µg/ml (durchschnittlicher $IC_{50}$-Wert) und 2,1 µg/ml (durchschnittlicher $IC_{70}$-Wert). Verglichen mit der Wirksamkeit von Standardchemotherapeutika in diesem Test liegen die Werte im Rahmen der $IC_{50}$ und $IC_{70}$-Werte der alkylierenden Mittel Ifosfamid und Cyclophosphamid. Die meisten anderen Standard-alkylierenden Mittel haben durchschnittliche $IC_{70}$-Werte von < 0,1 µg/ml.

Die sensitivsten Tumorxenotransplantate, repräsentiert durch $IC_{70}$-Werte und Balken nach links in Tabelle 3, waren das große Lungenzellkrebsxenotransplantat LXFL 529, das Nierenkrebsxenotransplantat RXF 393 und die Prostataxenotransplantate PC3M und DU145X.

## Tabelle 3

In vitro-Effekt von Natriumselenit auf menschliche Tumorxenotransplantate

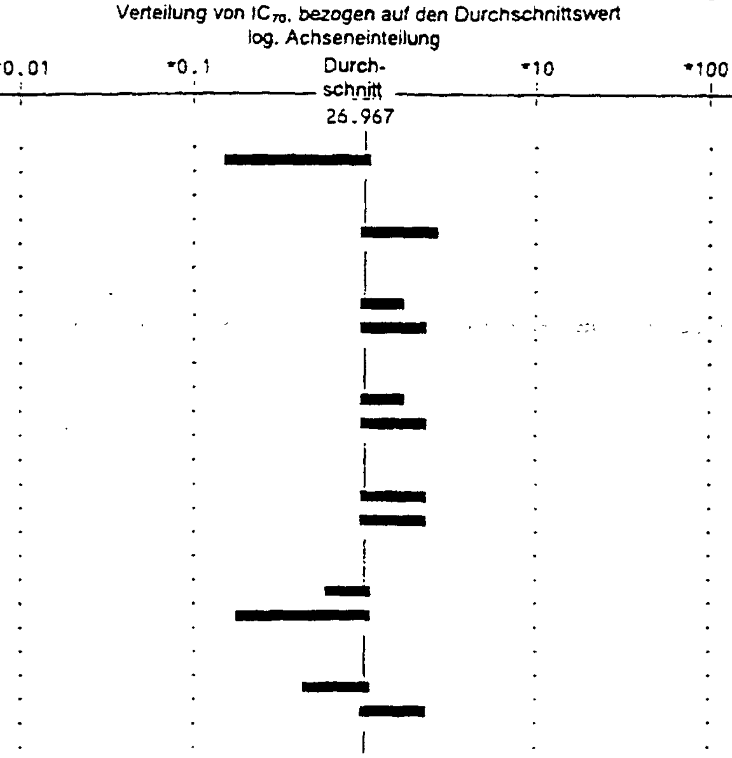


| TUMOR/ PASSAGE Nr. | Kolonie-Kontr. | Verteilung von $IC_{70}$, bezogen auf den Durchschnittswert log. Achseneinteilung | $IC_{50}$ µM | $IC_{70}$ µM |
|---|---|---|---|---|
| LXFL | | | | |
| 529/18 | 129 | | 1.627 | 3.945 |
| LXFS | | | | |
| 650/9 | 107 | | 43.093 | 70.706 |
| MAXF | | | | |
| 401/16 | 37 | | 18.873 | 41.753 |
| MCF7X/28 | 200 | | 30.516 | 49.059 |
| OVXF | | | | |
| 899/33 | 50 | | 20.866 | 39.556 |
| 1353/17 | 120 | | 31.622 | 50.118 |
| PAXF | | | | |
| 546/2 | 80 | | 27.660 | 47.251 |
| 736/17 | 55 | | 29.182 | 49.851 |
| PRXF | | | | |
| PC3M/3 | 140 | | 5.554 | 16.451 |
| DU145X/1 | 254 | | 2.997 | 5.120 |
| RXF | | | | |
| 393/9 | 23 | | n.e. | 12.328 |
| 423/16 | 97 | | 35.111 | 53.366 |
| Durchschnitt | n=12 | 26.967 | 15.5 | 27.0 |

2.2 In vitro Kombinationsstudien

Um zu untersuchen, ob Selen in der Form von Natriumselenit den anti-proliferativen Effekt von Standardchemotherapeutika potenzieren kann, wurden zwei pankreatische menschliche Tumorxenotransplantate (PAXF 546 und PAXF 736) Gemcitabin, Mitomycin C oder Natriumselenit alleine oder Natriumselenit in Kombination mit einem der beiden Chemotherapeutika ausgesetzt.

Festgelegte Konzentrationen an Natriumselenit (3 oder 30 µM) wurden den Zellen zusammen mit 6 verschiedenen Konzentrationen der Chemotherapeutika zugesetzt. Gemäß den in Tabelle 2 gezeigten Daten wurde erwartet, daß eine Natriumselenitkonzentration von 3 µM die Koloniebildung zweier pankreatischer Tumorzellinien nur marginal beeinflußt. Dies wurde durch die Ergebnisse bestätigt, wenn Natriumselenit alleine bei dieser Konzentration in den Kombinationsstudien eingesetzt wurde (Figur 1). Die höhere Natriumselenitkonzentration von 30 µM sollte einen stärkeren Einfluß auf die Tumorko-loniebildung haben, da die Tumorkoloniebildungsrate beider pankreatischer Tumoren schnell abfällt, wenn die Natriumselenitkonzentration von 10 auf 100 µM steigt (Tabelle 2). Dies wird in weiteren Experimenten in Figur 1 gezeigt. Die Abweichung der T/C-Werte bei 30 µM in den beiden Experimenten (Figur 1) wird durch die sehr steilen Dosis/Effekt-Werte von Natriumselenit bei Konzentrationen um 30 µM verursacht. Daher wurden Kombinationsexperimente mit PAXF 546 bei dieser Selenkonzentration getrennt bewertet. Im Falle von PAXF 736 konnte lediglich Experiment X265 für die Bewertung herangezogen werden, da in Experiment X290 30 µM an Selen bereits einen stark zytotoxischen Effekt mit einem T/C-Wert von 5% zeigten.

Die folgenden Tabellen 4a bis 4d zeigen die Ergebnisse der Inkubation zweier pankreatischer Xenotransplantate mit verschiedenen Konzentrationen an Selen und den Zytostatika Mitomycin C und Gemcitabin. Um zu bestimmen, ob eine Wirkstoffkombination einen additiven Effekt der Einzelwirkstoffe aufweist, wurden die experimentell ermittelten T/C-Werte für eine spezifische Kombination mit den Werten, die für einen additiven Effekt erwartet wurden, verglichen (siehe Material und Methoden). Wenn das pankreatische Tumorxenotransplantat PAXF 736 gleichzeitig mit Natriumselenit und Mitomycin C oder Gemcitabin behandelt wurde, konnte weder bei einer Konzentration von 3 µM noch bei einer Konzentration von 30 µM Natriumselenit ein synergistischer Effekt festgestellt werden. Es wurde ebenfalls kein

synergistischer Effekt von 3 µM Natriumselenit und allen der getesteten Konzentrationen an Chemotherapeutika in dem Xenotransplantat PAXF 546 beobachtet oder wenn geringe Konzentrationen der Chemotherapeutika mit 30 µM an Natriumselenit kombiniert wurden. Wurden jedoch höhere Dosierungen der Chemotherapeutika, bei denen zytotoxische Effekte beobachtet werden konnten, zusammen mit 30 µM Natriumselenit eingesetzt, wurde ein Synergismus in Kombination mit Mitomycin C und Gemcitabin beobachtet (Tabelle 4a, 4b).

## Tabelle 4a

Inhibition von Koloniewachstum des Pankreaskrebs-Xenotransplantates PAXF 546 durch $Na_2SeO_3$ oder Mitomycin C alleine oder in Kombination *in vitro*

| Behandlung | | Natriumselenit Konz. in µM | Natriumselenit T/C in % | T/C in % bei einer Mitomycin C Konzentration (µM) von: $1x10^{6}$ | $1x10^{-5}$ | $1x10^{-4}$ | $1x10^{-3}$ | $1x10^{-2}$ | $1x10^{-1}$ | 1.0 | Synergismus |
|---|---|---|---|---|---|---|---|---|---|---|---|
| Mitomycin C | (X261, X295) | | | **87 ± 11** | **84 ± 14** | **70 ± 10** | **70 ± 13** | **80 ± 12** | **57 ± 8** | **5 ± 2** | |
| Mitomycin C + 3 µM Se | erwart. beob.* (X261, X295) | 3.0 | **91 ± 8** | 84 ± 15 | 84 ± 13 | 72 ± 12 | 66 ± 10 | 60 ± 8 | 60 ± 10 | 4 ± 2 | nein |
| | erwartet** | | | 76 ± 12 | 76 ± 15 | 64 ± 11 | 64 ± 14 | 73 ± 13 | 52 ± 9 | 5 ± 3 | |
| Mitomycin C + 30 µM Se | erwart. beob. (X261) | 30.0 | **80 ± 13** | 69 ± 10 | 58 ± 8 | 49 ± 7 | 50 ± 8 | ***35 ± 6**** | ***3 ± 2*** | | partiell |
| | erwartet | | | 70 ± 14 | 67 ± 16 | 56 ± 14 | 56 ± 15 | ***64 ± 14*** | ***46 ± 10*** | | |
| | erwart. beob. (X295) | 30.0 | **49 ± 6** | 38 ± 8 | 37 ± 6 | 31 ± 5 | 30 ± 6 | ***26 ± 5*** | ***14 ± 3*** | | partiell |
| | erwartet | | | 43 ± 12 | 41 ± 15 | 34 ± 11 | 34 ± 14 | ***39 ± 12*** | ***28 ± 9*** | | |

* experimentell beobachtete T/C-Werte für die Kombination von Mitomycin C und Selen

** erwartete T/C-Werte für die Kombination, errechnet durch das Verfahren: $T/C_{erwartet} = T/C_{se} \times T/C_{Wirkstoff}/100$; T/C: T/C-Wert in % bei einer gegebenen Behandlung bei einer spezifischen Konzentration

*** synergistische Wirkstoffkonzentrationen sind durch Kursivdruck gekennzeichnet

## Tabelle 4b

Inhibition von Koloniewachstum des Pankreaskrebs-Xenotransplantates PAXF 546 durch $Na_2SeO_3$ oder Gemcitabin alleine oder in Kombination *in vitro*

| Behandlung | | Natriumselenit: Konz. in µM | Natriumselenit: T/C in % | T/C in % bei einer Gemcitabin Konzentration (µM) von: 1x10⁻⁶ | 1x10⁻⁵ | 1x10⁻⁴ | 1x10⁻³ | 1x10⁻² | 1x10⁻¹ | 1.0 | Synergismus |
|---|---|---|---|---|---|---|---|---|---|---|---|
| Gemcitabin | (X261, X295) | | | **85 ± 15** | **73 ± 20** | **80 ± 12** | **83 ± 12** | **61 ± 11** | **48 ± 8** | **30 ± 6** | |
| Gemcitabin + 3 µM Se | erwart. beob.* (X261, X295) | 3.0 | **91 ± 8** | 86 ± 16 | 93 ± 16 | 70 ± 18 | 72 ± 11 | 50 ± 9 | 41 ± 10 | 30 ± 10 | nein |
| | erwartet** | | | 77 ± 17 | 66 ± 23 | 73 ± 14 | 76 ± 14 | 56 ± 12 | 44 ± 10 | 34 ± 8 | |
| Gemcitabin + 30 µM Se | erwart. beob. (X261) | 30.0 | **80 ± 13** | 74 ± 12 | 57 ± 7 | 58 ± 7 | *40 ± 8**** | *24 ± 7* | *27 ± 4* | | partiell |
| | erwartet | | | 68 ± 17 | 58 ± 23 | 64 ± 15 | *66 ± 15* | *49 ± 14* | *38 ± 12* | | |
| | erwart. beob. (X295) | 30.0 | **49 ± 6** | 55 ±12 | 41 ± 10 | 50 ± 9 | *23 ± 7* | 23 ± 6 | *11 ± 6* | *10 ± 3* | partiell |
| | erwartet | | | 43 ± 14 | 36 ± 21 | 39 ± 13 | *41 ± 13* | 30 ± 12 | *24 ± 9* | *18 ± 7* | |

* experimentell beobachtete T/C-Werte für die Kombination von Gemcitabin und Selen

** erwartete T/C-Werte für die Kombination, errechnet durch das Verfahren: $T/C_{erwartet} = T/C_{se} \times T/C_{Wirkstoff}/100$; T/C: T/C-Wert in % bei einer gegebenen Behandlung bei einer spezifischen Konzentration

*** synergistische Wirkstoffkonzentrationen sind durch Kursivdruck gekennzeichnet

## Tabelle 4c

Inhibition von Koloniewachstum des Pankreaskrebs-Xenotransplantates PAXF 736 durch $Na_2SeO_3$ oder Mitomycin C alleine oder in Kombination *in vitro*

| Behandlung | | Natriumselenit | | T/C in % bei einer Mitomycin C Konzentration von | | | | | | Synergismus |
|---|---|---|---|---|---|---|---|---|---|---|
| | | Konz. in µM | T/C in % | $1x10^{-6}$ | $1x10^{5}$ | $1x10^{-4}$ | $1x10^{-3}$ | $1x10^{-2}$ | $1x10^{-1}$ | |
| Mitomycin C | (X261, X295) | | | 77 ± 13 | 58 ± 10 | 56 ± 11 | 46 ± 8 | 30 ± 7 | **11 ± 7** | |
| Mitomycin C + 3 µM Se | erwart. beob.* (X265, X290) | 3.0 | 81 ± 12 | 90 ± 15 | 60 ± 11 | 56 ± 8 | 48 ± 7 | 29 ± 3 | 10 ± 3 | nein |
| | erwartet** | | | 70 ± 16 | 53 ± 13 | 51 ± 13 | 42 ± 12 | 27 ± 9 | 10 ± 9 | |
| Mitomycin C + 30 µM Se | erwart. beob. (X265) | 30.0 | **43 ± 8** | 25 ± 10 | 15 ± 7 | 27 ± 7 | 17 ± 6 | 19 ± 6 | 4 ± 3 | nein |
| | erwartet | | | 33 ± 14 | 25 ± 11 | 24 ± 12 | 20 ± 9 | 14 ± 8 | 5 ± 5 | |
| | erwart. beob. (X290) | 30.0 | **5 ± 2** | 4 ± 2 | 3 ± 2 | 3 ± 3 | 2 ± 2 | 1 ± 1 | 1 ± 1 | n.b.*** |
| | erwartet ı | | | | | | | | | |

* experimentell beobachtete T/C-Werte für die Kombination von Mitomycin C und Selen

** erwartete T/C-Werte für die Kombination, errechnet durch das Multiplikationsverfahren: $T/C_{erwartet} = T/C_{se} \times T/C_{Wirkstoff}/100$; T/C: T/C-Wert in % bei einer gegebenen Behandlung bei einer spezifischen Konzentration

*** nicht bestimmt

**Tabelle 4d**

Inhibition von Koloniewachstum des Pankreaskrebs-Xenotransplantates PAXF 736 durch $Na_2SeO_3$ oder Gemcitabin alleine oder in Kombination *in vitro*

| Behandlung | | Natriumselenit Konz. in µM | Natriumselenit T/C in % | T/C in % bei einer Gemcitabin Konzentration (µM) von | | | | | | Synergismus |
|---|---|---|---|---|---|---|---|---|---|---|
| | | | | $1x10^{6}$ | $1x10^{5}$ | $1x10^{-4}$ | $1x10^{-3}$ | $1x10^{-2}$ | $1x10^{-1}$ | |
| Gemcitabin | (X261, X295) | | | **89 ± 11** | **78 ± 14** | **84 ± 12** | **77 ± 13** | **71 ± 10** | **30 ± 8** | |
| Gemcitabin + 3 µM Se | erwart. beob.* (X265, X290) | 3.0 | **81 ± 12** | 79 ± 13 | 82 ± 12 | 84 ± 12 | 86 ± 15 | 66 ± 10 | 18 ± 8 | nein |
| | erwartet** | | | 72 ± 12 | 63 ± 16 | 68 ± 13 | 62 ± 14 | 58 ± 12 | 24 ± 9 | |
| Gemcitabin + 30 µM Se | erwart. beob. (X265) | 30.0 | **43 ± 8** | 64 ± 15 | 62 ± 20 | 59 ± 13 | 39 ± 7 | 34 ± 6 | 14 ± 5 | nein |
| | erwartet | | | 38 ± 12 | 34 ± 15 | 36 ± 13 | 33 ± 14 | 31 ± 11 | 13 ± 9 | |
| | erwart. beob. (X290) | 30.0 | **5 ± 2** | 9 ± 7 | 5 ± 3 | 4 ± 3 | 5 ± 3 | 3 ± 2 | 1 ± 1 | n.b.*** |
| | erwartet | | | | | | | | | |

* experimentell beobachtete T/C-Werte für die Kombination von Gemcitabin und Selen

** erwartete T/C-Werte für die Kombination, errechnet durch das Multiplikationsverfahren: $T/C_{erwartet} = T/C_{se} \times T/C_{Wirkstoff}/100$; T/C: T/C-Wert in % bei einer gegebenen Behandlung bei einer spezifischen Konzentration

*** nicht bestimmt

Tabelle 5a:

| Synergistische Effekte von Selen in Kombination mit Gemcitabin in vitro | | | | | | | | | |
|---|---|---|---|---|---|---|---|---|---|
| Xenotransplantat | Exp. Nr. | Se Konz. [µM] | Synergismus mit Gemcitabin [µM] | | | | | | |
| | | | $1\times10^{-6}$ | $1\times10^{-5}$ | $1\times10^{-4}$ | $1\times10^{-3}$ | $1\times10^{-2}$ | 0.1 | 1.0 |
| PAXF 546 | X261, X295 | 3.0 | - | - | - | - | - | - | - |
| | X261 | 30.0 | - | - | - | + | + | - | n. b. |
| | X295 | 30.0 | - | - | - | + | + | + | + |
| PAXF 736 | X265, X290 | 3.0 | - | - | - | - | - | - | n. b. |
| | X265 | 30.0 | - | - | - | - | - | - | n. b. |
| | X290 | 30.0 | nicht bestimmt | | | | | | |

Tabelle 5b:

| Synergistische Effekte von Selen in Kombination mit Mitomycin C in vitro | | | | | | | | | |
|---|---|---|---|---|---|---|---|---|---|
| Xenotransplantat | Exp. Nr. | Se Konz. [µM] | Synergismus mit Mitomycin C [µM] | | | | | | |
| | | | $1x10^{-6}$ | $1x10^{-5}$ | $1x10^{-4}$ | $1x10^{-3}$ | $1\times10^{-2}$ | 0.1 | 1.0 |
| PAXF 546 | X261, X295 | 3.0 | - | - | - | - | - | - | - |
| | X261 | 30.0 | - | - | - | - | + | + | n.b. |
| | X295 | 30.0 | - | - | - | - | + | + | + |
| PAXF 736 | X265, X290 | 3.0 | - | - | - | - | - | - | n. b. |
| | X265 | 30.0 | - | - | - | - | - | - | n.b. |
| | X290 | 30.0 | nicht bestimmt | | | | | | |
| * Der experimentell bestimmte T/C-Wert für eine Wirkstoffkombination ist deutlich geringer (+) oder nicht geringer (-) als der erwartete T/C-Wert für diese Kombination: n.b.: nicht bestimmt. | | | | | | | | | |

In Figuren 2 und 3 ist der Effekt von Natriumselenit und Mitomycin C auf das in vitro-Wachstum von PAXF 546 dargestellt. Wenn 3 µM Natriumselenit zu verschiedenen Konzentrationen an Mitomycin C zugesetzt wurden, konnte keine Änderung in der Dosis/Wirkungskurve von Mitomycin C beobachtet werden, woraus folgert, daß bei dieser niedrigen Konzentration an Selenit kein Synergismus zwischen den beiden Wirkstoffen auftritt (Figuren 2a und 3a).

Wurden jedoch 30 µM Natriumselenit gleichzeitig mit Mitomycin C verabreicht, wurde ein synergistischer Effekt bei Mitomycin C-Konzentrationen > 0,01 µM beobachtet (Figuren 2b, c, 3b, c und Tabelle 5b).

Ähnliche Ergebnisse wurden mit der Kombination von Natriumselenit und Gemcitabin erzielt (Figur 4 und 5, Tabelle 5a). Eine synergistische Inhibition der Koloniebildung in PAXF 546 wurde ebenfalls bei einer Selenkonzentration von 30 µM und einer Gemcitabin-Konzentration höher als 0,1 nM festgestellt (Figur 5, Tabelle 5a).

Zusammenfassend zeigen die Ergebnisse, daß hohe Dosen an Selen das Wachstum vieler menschlicher Tumorxenotransplantate in vitro reduzieren. Gleichzeitige Behandlung der pankreatischen Krebszellen PAXF 546 mit 30 µM Selen und Zytostatika, weiche direkt mit der zellulären DNA (z.B Gemcitabin und Mitomycin C) reagieren, resultiert in einer synergistischen Inhibierung des Tumorwachstums in vitro.

**3. Fallstudien**

Im folgenden sind die Falldokumentationen von fünf männlichen Tumorpatienten, die eine Kombinationstherapie von hochdosiertem Selen (10-30 mg) und verschiedenen Zytostatika erhielten, aufgeführt. Zwei der Patienten mit Pankreaskarzinom (Patient Nr. 4, 5) zeigten trotz weit fortgeschrittener Erkrankung ein gutes Ansprechen auf die

Therapie, ebenso wie zwei Patienten (Patient Nr. 1, 3) mit hormonresistenten metastasierten Prostatakarzinomen. Ein Patient (Patient Nr. 2) mit metastasiertem Hypernephrom reagierte mit einer Teilremission auf die Therapie.

Patient Nr. 1

1.) Diagnose: hormonresistentes metastasiertes Prostatakarzinom

2.) Diagnose gestellt (Monat/Jahr): Juni 1994

3.) Histologie:cribriformes Prostatakarzinom, Grad III

4.) Tumorstadium zum Zeitpunkt des Therapiebeginns: T3, N2, M1
(T = Ausdehnung des Primärtumors
N = Vorhandensein von Lymphknotenmetastasen
M = Vorhandensein von Fernmetastasen)

5.) Lokalisation der Fernmetastasen: Lymphknoten, Knochen

6.) Tumorvorbehandlung: Hormontherapie von VI/94 bis XI/94

7.) Therapie mit Chemotherapie/Selen:

a) Chemotherapeutikum: 5-Fluoruracil, 750 mg, i.v., von XI/94 bis V/95 Mitomycin, 10 mg, i.v., von XI/94 bis V/95
b) Selen: Selenase®, 10 mg, i.v., von XI/94 bis V/95
c) Ansprechen der Therapie: Teilremission, vollständiger Rückgang der Lymph ödeme
d) Dauer des Ansprechens: 7 Monate
e) Grund für Beendigung der Therapie: Progression
f) Verträglichkeit der Therapie: gut

8.) Vor- und Begleiterkrankungen: koronale Herzkrankheit seit 1991, bei Therapiebeginn noch bestehend

9.) Begleittherapie während Chemo/Selen-Therapie: Kerlone, Adalat von 1991 bis V/95

10.) Überlebensstatus: tot
Todesdatum: 31.08.1994; tumorbedingt

Patient Nr. 2

1.) Diagnose: metastasiertes Hypernephrom

2.) Diagnose gestellt (Monat/Jahr): April 1996

3.) Histologie: Hypernephrom

4) Tumorstadium zum Zeitpunkt des Therapiebeginns: T2, N1, M1
(T = Ausdehnung des Primärtumors
N = Vorhandensein von Lymphknotenmetastasen
M = Vorhandensein von Fernmetastasen)

5.) Lokalisation der Fernmetastasen: Lunge, Leber

6.) Tumorvorbehandlung:

Nephrektomie 1996; Interleukin-II von IX/97 bis I/98

7.) Therapie mit Chemotherapie/Selen

a) Chemotherapeutikum: Gemzar, 2 g, i.v., von I/98 bis V/98
b) Selen: Selenase®, 30 mg, i.v von I/98 bis V/98
c) Ansprechen der Therapie: Teilremission
d) Dauer des Ansprechens: bislang 5 Monate
e) Grund für Beendigung der Therapie: keiner
f) Verträglichkeit der Therapie: gut

8.) Vor- und Begleiterkrankungen: Polyzythämie seit 1990; bei Therapiebeginn noch bestehend

9.) Begleittherapie während Chemo/Selen-Therapie: keine

10.) Überlebensstatus: lebend; zuletzt beobachtet 18.05.1998

Patient Nr. 3

1.) Diagnose: metastasiertes hormonresistentes Prostatakarzinom

2.) Diagnose gestellt (Monat/Jahr): Mai 1997

3.) Histologie: Adenokarzinom, Grad II

4:) Tumorstadium zum Zeitpunkt des Therapiebeginns: T3, N1, M1
(T = Ausdehnung des Primärtumors
N = Vorhandensein von Lymphknotenmetastasen
M = Vorhandensein von Fernmetastasen)

5.) Lokalisation der Fernmetastasen: Knochen

6.) Tumorvorbehandlung:Orchiektomie beiderseits 1997

7.) Therapie mit Chemotherapie/Selen:

a) Chemotherapeutikum: Adriblastin, 40 mg, i.v., von II/98 bis V/98
b) Selen: Selenase®, 30 mg, i.v., von II/98 bis V/98
c) Ansprechen der Therapie: Vollremission
d) Dauer des Ansprechens: bislang 3 Monate
e) Grund für Beendigung der Therapie: keiner
f) Verträglichkeit der Therapie: gut

8.) Vor- und Begleiterkrankungen: keine

9.) Begleittherapie während Chemo/Selen-Therapie: Bisphosphonat (Bondronat) von II/98 bis V/98

10.) Überlebensstatus: lebend; zuletzt beobachtet 20.05.1998

Patient Nr. 4

1.) Diagnose: Pankreaskarzinom

2.) Diagnose gestellt (Monat/Jahr): Oktober 1994

3.) Histologie: Adenokarzinom. Grad II

4.) Tumorstadium zum Zeitpunkt des Therapiebeginns: T4, N1, M0
(T = Ausdehnung des Primärtumors
N = Vorhandensein von Lymphknotenmetastasen
M = Vorhandensein von Fernmetastasen)

5.) Lokalisation der Fernmetastasen: keine

6.) Tumorvorbehandlung: explorative Laparotomie 1994, Gastroenterostomie

7.) Therapie mit Chemotherapie/Selen:

a) Chemotherapeutikum: 5-Fluoruracil, 750 mg, i.v.. von X/94 bis VI/95, Mitomycin, 10 mg, i.v., von X/94 bis VI/95
b) Selen: Selenase® 10 mg, i.v., von X/94 bis VI/95
c) Ansprechen der Therapie: Vollremission, Schmerzfreiheit, Gewichtszunahme
d) Dauer des Ansprechens: 9 Monate
e) Grund für Beendigung der Therapie: VI/95 Hirnmetastase
f) Verträglichkeit der Therapie: gut

8.) Vor- und Begleiterkrankungen: keine

9.) Begleittherapie während Chemo/Selen-Therapie: keine

10.) Überlebensstatus: Todesdatum 29.08.1995; tumorbedingt

<u>Patient Nr. 5</u>

1.) Diagnose: Pankreaskarzinom und Magenkarzinom

2.) Diagnose gestellt (Monat/Jahr): November 1997

3.) Histologie: Adenokarzinom, Grad III

4 ) Tumorstadium zum Zeitpunkt des Therapiebeginns T4, N1, M1
(T = Ausdehnung des Primärtumors
N = Vorhandensein von Lymphknotenmetastasen
M = Vorhandensein von Fernmetastasen)

5.) Lokalisation der Fernmetastasen: Leber, Lymphknoten

6.) Tumorvorbehandlung:

a) Operation: Probelaparotomie, Gastroenterostomie 1997
b) Chemotherapie: Gemza von XI/97 bis I/98, Hochdosis-5-Fluoruracil und Leukovorine von I/98 bis III/98, Oxaliplatin von III/98 bis IV/98

7.) Therapie mit Chemotherapie/Selen:

a) Chemotherapeutikum: Gemzar, 1,2 mg, i.v., von IV/98 bis V/98 Mitomycin, 10 mg, i.v. von IV/98 bis V/98
b) Selen: Selenase®, 30 mg, i.v., von IV/98 bis V/98
c) Ansprechen der Therapie: Teilremission, erhebliche Schmerzreduktion
d) Dauer des Ansprechens: 1 Monat
e) Grund für Beendigung der Therapie: Tod
f) Verträglichkeit der Therapie: ausgeprägte Stomatitis

8.) Vor- und Begleiterkrankungen: keine

9.) Begleittherapie während Chemo/Selen-Therapie: keine

10.) Überlebensstatus: tot
Todesdatum: 13.05.1998;
Todesursache: Panzytopenie

## Patentansprüche

1. Verwendung von Selen und/oder mindestens einer Selenverbindung zur Wirkungssteigerung des Zytostatikums Gemcitabin oder des Zytostatikums Mitomycin C.

2. Verwendung nach Anspruch 1, **dadurch gekennzeichnet, dass** die Selenverbindung eine organische Selenverbindung ist.

3. Verwendung nach Anspruch 2, **dadurch gekennzeichnet, dass** die organische Selenverbindung Selenomethionin ist.

4. Verwendung nach Anspruch 2, **dadurch gekennzeichnet, dass** die organische Selenverbindung Selenocystein ist.

5. Verwendung nach Anspruch 2, **dadurch gekennzeichnet, dass** die organische Selenverbindung Phenylenbis (methylen)selenocyanat ist.

6. Verwendung nach Anspruch 1, **dadurch gekennzeichnet, dass** die Selenverbindung ein Selenoxid ist.

7. Verwendung nach Anspruch 6, **dadurch gekennzeichnet, dass** die Selenverbindung ein Salz von $SeO_2$ ist.

8. Verwendung von Selen und/oder mindestens einer selenhaltigen Verbindung in Kombination mit dem Zytostatikum Gemcitabin oder mit dem Zytostatikum Mitomycin C zur Herstellung eines Medikaments zur Behandlung von Krebs, **dadurch gekennzeichnet, dass** Selen und/oder die Selenverbindung in einer Konzentration von 0,1 mg/kg Körpergewicht bis 1,25 mg/kg Körpergewicht, und das Zytostatikum in einer Konzentration von 2 $mg/m^2$ Körperoberfläche bis 240 $g/m^2$ Körperoberfläche zu verwendet ist.

9. Verwendung nach Anspruch 8, **dadurch gekennzeichnet, dass** Selen und/oder die Selenverbindung in einer Konzentration von 0,1 mg/kg Körpergewicht bis 0,3 mg/kg Körpergewicht, und das Zytostatikum in einer Konzentration von 20 $mg/m^2$ Körperoberfläche bis 1000 $mg/m^2$ Körperoberfläche zu verwenden ist.

10. Verwendung nach Anspruch 8 oder 9, **dadurch gekennzeichnet, dass** die Selenverbindung eine organische Selenverbindung ist.

11. Verwendung nach Anspruch 10, **dadurch gekennzeichnet, dass** die organische Selenverbindung Selenomethionin ist.

12. Verwendung nach Anspruch 10, **dadurch gekennzeichnet, dass** die organische Selenverbindung Selenocystein ist.

13. Verwendung nach Anspruch 10, **dadurch gekennzeichnet, dass** die organische Selenverbindung Phenylenbis (methylen)selenocyanat ist.

14. Verwendung nach Anspruch 8 oder 9, **dadurch gekennzeichnet, dass** die Selenverbindung ein Selenoxid ist.

15. Verwendung nach mindestens einem der Anspruch 14, **dadurch gekennzeichnet, dass** die Selenverbindung ein Salz von $SeO_2$ ist.

16. Kit, umfassend

a) Selen und/oder mindestens eine Selenverbindung und
b) das Zytostatikum Gemcitabin oder das Zytostatikum Mitomycin C,

als Kombinationspräparat zur gleichzeitigen, getrennten oder zeitlich abgestuften Anwendung in der zytostatischen Therapie, **dadurch gekennzeichnet, dass** Selen und/oder die Selenverbindung in einer Konzentration von 0,1 mg/kg Körpergewicht bis 1,25 mg/kg Körpergewicht, und das Zytostatikum in einer Konzentration von 2 $mg/m^2$ Körperoberfläche bis 240 $g/m^2$ Körperoberfläche verwendet wird.

**17.** Kit nach Anspruch 16, **dadurch gekennzeichnet, dass** Selen und/oder die Selenverbindung in einer Konzentration von 0,1 mg/kg Körpergewicht bis 0,3 mg/kg Körpergewicht, und das Zytostatikum in einer Konzentration von 20 $mg/m^2$ Körperoberfläche bis 1000 $mg/m^2$ Körperoberfläche verwendet wird.

**18.** Kit nach Anspruch 16 oder 17, **dadurch gekennzeichnet, dass** die Selenverbindung eine organische Selenverbindung ist.

**19.** Kit nach Anspruch 18, **dadurch gekennzeichnet, dass** die organische Selenverbindung Selenomethionin ist.

**20.** Kit nach Anspruch 18, **dadurch gekennzeichnet, dass** die organische Selenverbindung Selenocystein ist.

**21.** Kit nach Anspruch 18, **dadurch gekennzeichnet, dass** die organische Selenverbindung Phenylenbis(methylen) selenocyanat ist.

**22.** Kit nach Anspruch 16 oder 17, **dadurch gekennzeichnet, dass** die Selenverbindung ein Selenoxid ist.

**23.** Kit nach Anspruch 22, **dadurch gekennzeichnet, dass** die Selenverbindung ein Salz von $SeO_2$ ist.

## Claims

**1.** Use of selenium and/or at least one selenium compound for enhancing the effect of the cytostatic Gemcitabine or the cytostatic Mitomycin C.

**2.** Use according to claim 1, **characterized in that** the selenium compound is an organic selenium compound.

**3.** Use according to claim 2, **characterized in that** the organic selenium compound is selenium methionine.

**4.** Use according to claim 2, **characterized in that** the organic selenium compound is selenocysteine.

**5.** Use according to claim 2, **characterized in that** the organic selenium compound is phenylenebis(methylene) selenocyanate.

**6.** Use according to claim 1, **characterized in that** the selenium compound is selenium oxide.

**7.** Use according to claim 1 or 6, **characterized in that** the selenium compound is a salt of $SeO_2$.

**8.** Use of selenium and/or at least one selenium compound in combination with the cytostatic Gemcitabine or the cytostatic Mitomycin C for the preparation of a medicament for the treatment of cancer, **characterized in that** selenium and/or the selenium compound is used in a concentration of 0.1 mg/kg body weight to 1.25 mg/kg body weight, and the cystostatic is used in a concentration of 2 $mg/m^2$ body surface to 240 $g/m^2$ body surface.

**9.** Use according to claim 8, **characterized in that** selenium and/or the selenium compound is used in a concentration of 0.1 mg/kg body weight to 0.3 mg/kg body weight, and the cytostatic is used in a concentration of 20 $mg/m^2$ body surface to 1000 $mg/m^2$ body surface.

**10.** Use according to claim 8 or 9, **characterized in that** the selenium compound is an organic selenium compound.

**11.** Use according to claim 10, **characterized in that** the organic selenium compound is selenomethionine.

12. Use according to claim 10, **characterized in that** the organic selenium compound is selenocysteine.

13. Use according to claim 10, **characterized in that** the organic selenium compound is phenylenebis(methylene) selenocyanate.

14. Use according to claim 8 or 9, **characterized in that** the selenium compound is selenium oxide.

15. Use according to claim 14, **characterized in that** the selenium compound is a salt of $SeO_2$.

16. Kit comprising

a) selenium and/or at least one selenium compound; and

b) the cytostatic Gemcitabine or the cytostatic Mitomycin C, as a combination preparation for simultaneous, separate or sequential application in cytostatic therapy,

**characterized in that** the selenium and/or the selenium compound is used in a concentration of 0.1 mg/kg body weight to 1,25 mg/kg body weight and the cytostatic is used in a concentration of 20 $mg/m^2$ body surface to 240 $mg/m^2$ body surface.

17. Kit according to claim 16, **characterized in that** the selenium and/or the selenium compound is used in a concentration of 0.1 mg/kg body weight to 0.3 mg/kg body weight and the cytostatic is used in a concentration of 20 $mg/m^2$ body surface to 1000 $mg/m^2$ body surface.

18. Kit according to claim 16 or 17, **characterized in that** the selenium compound is an organic selenium compound.

19. Kit according to claim 18, **characterized in that** the organic selenium compound is selenomethionine.

20. Kit according to claim 18, **characterized in that** the organic selenium compound is selenocysteine.

21. Kit according to claim 18, **characterized in that** the organic selenium compound is phenylenebis(methylene) selenocyanate.

22. Kit according to claim 16 or 17, **characterized in that** the selenium compound is selenium oxide.

23. Kit according to claim 22, **characteized in** that the organic selenium compound is salt of $SeO_2$.

## Revendications

1. Utilisation du sélénium et/ou d'au moins un composé du sélénium pour augmenter l'action du cytostatique gemcitabine ou du cytostatique mitomycine C.

2. Utilisation selon la revendication 1, **caractérisée en ce que** le composé du sélénium est un composé organique du sélénium.

3. Utilisation selon la revendication 2, **caractérisée en ce que** le composé organique du sélénium est la sélénométhionine.

4. Utilisation selon la revendication 2, **caractérisée en ce que** le composé organique du sélénium est la sélénocystéine.

5. Utilisation selon la revendication 2, **caractérisée en ce que** le composé organique du sélénium est le bis(méthylène)sélénocyanate de phénylène.

6. Utilisation selon la revendication 1, **caractérisée en ce que** le composé du sélénium est un oxyde de sélénium.

7. Utilisation selon la revendication 6, **caractérisée en ce que** le composé du sélénium est un sel de $SeO_2$.

**8.** Utilisation du sélénium et/ou d'au moins un composé comprenant du sélénium en combinaison avec le cytostatique gemcitabine ou avec le cytostatique mitomycine C pour la fabrication d'un médicament pour le traitement du cancer, **caractérisée en ce que** le sélénium et/ou le composé du sélénium est utilisé à une concentration de 0,1 mg/kg de poids corporel à 1,25 mg/kg de poids corporel, et **en ce que** le cytostatique est utilisé à une concentration de 2 $mg/m^2$ de surface corporelle à 240 $g/m^2$ de surface corporelle.

**9.** Utilisation selon la revendication 8, **caractérisée en ce que** le sélénium et/ou le composé du sélénium est utilisé à une concentration de 0,1 mg/kg de poids corporel à 0,3 mg/kg de poids corporel, et **en ce que** le cytostatique est utilisé à une concentration de 20 $mg/m^2$ de surface corporelle à 1000 $mg/m^2$ de surface corporelle.

**10.** Utilisation selon la revendication 8 ou 9, **caractérisée en ce que** le composé du sélénium est un composé organique du sélénium.

**11.** Utilisation selon la revendication 10, **caractérisée en ce que** le composé organique du sélénium est la sélénométhionine.

**12.** Utilisation selon la revendication 10, **caractérisée en ce que** le composé organique du sélénium est la sélénocystéine.

**13.** Utilisation selon la revendication 10, **caractérisée en ce que** le composé organique du sélénium est le bis(méthylène)sélénocyanate de phénylène.

**14.** Utilisation selon la revendication 8 ou 9, **caractérisée en ce que** le composé du sélénium est un oxyde de sélénium.

**15.** Utilisation selon la revendication 14, **caractérisée en ce que** le composé du sélénium est un sel de $SeO_2$.

**16.** Trousse comprenant

a) du sélénium et/ou au moins un composé du sélénium et
b) le cytostatique gemcitabine ou le cytostatique mitomycine C,

en tant que préparation combinée pour une mise en oeuvre simultanée, séparée ou échelonnée dans le temps dans la thérapie cytostatique, **caractérisée en ce que** le sélénium et/ou le composé du sélénium est utilisé à une concentration de 0,1 mg/kg de poids corporel à 1,25 mg/kg de poids corporel, et **en ce que** le cytostatique est utilisé à une concentration de 2 $mg/m^2$ de surface corporelle à 240 $g/m^2$ de surface corporelle.

**17.** Trousse selon la revendication 16, **caractérisée en ce que** le sélénium et/ou le composé du sélénium est utilisé à une concentration de 0,1 mg/kg de poids corporel à 0,3 mg/kg de poids corporel, et **en ce que** le cytostatique est utilisé à une concentration de 20 $mg/m^2$ de surface corporelle à 1000 $mg/m^2$ de surface corporelle.

**18.** Trousse selon la revendication 16 ou 17, **caractérisée en ce que** le composé du sélénium est un composé organique du sélénium.

**19.** Trousse selon la revendication 18, **caractérisée en ce que** le composé organique du sélénium est la sélénométhionine.

**20.** Trousse selon la revendication 18, **caractérisée en ce que** le composé organique du sélénium est la sélénocystéine.

**21.** Trousse selon la revendication 18, **caractérisée en ce que** le composé organique du sélénium est le bis(méthylène)sélénocyanate de phénylène.

**22.** Trousse selon la revendication 16 ou 17, **caractérisée en ce que** le composé du sélénium est un oxyde de sélénium.

**23.** Trousse selon la revendication 22, **caractérisée en ce que** le composé du sélénium est un sel de $SeO_2$.

*Fig. 1*

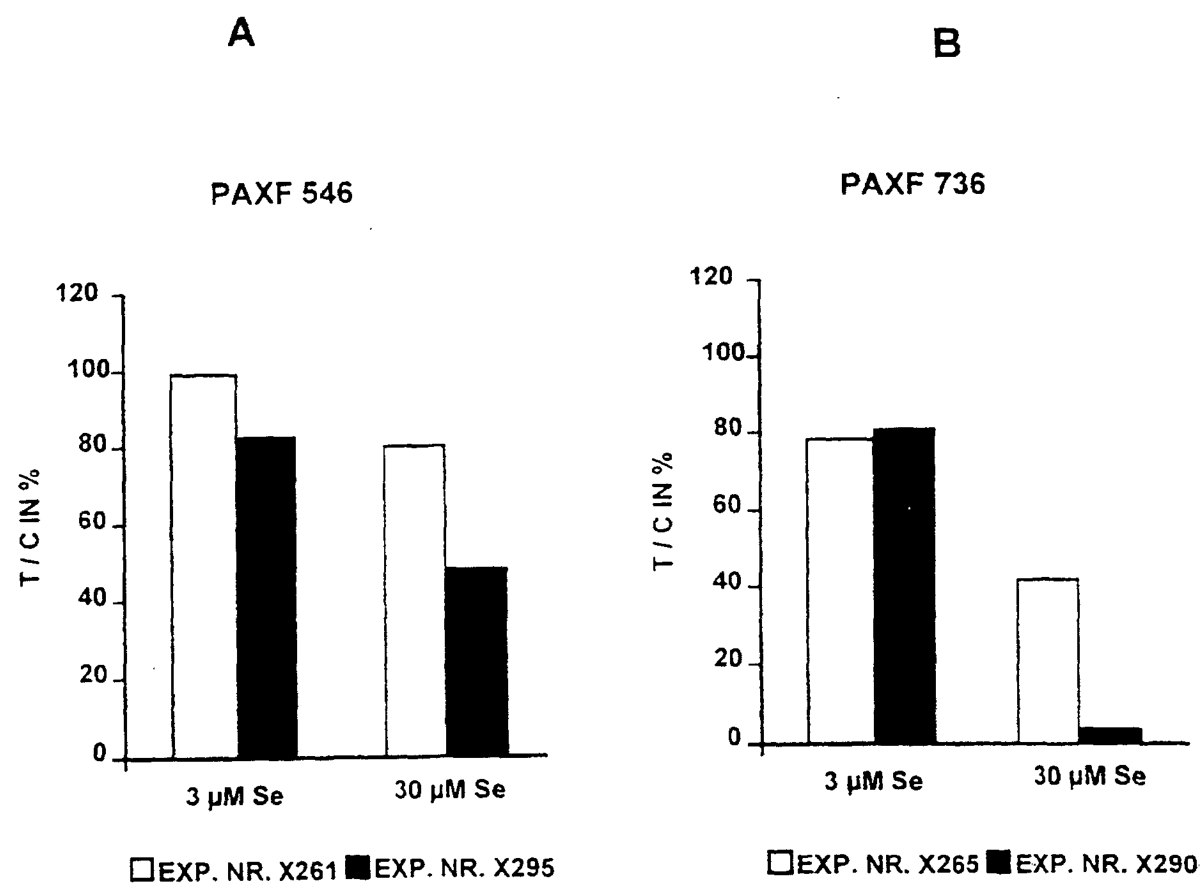
A
B
PAXF 546
PAXF 736
T / C IN %
120
100
80
60
40
20
0
3 µM Se
30 µM Se
EXP. NR. X261
EXP. NR. X295
EXP. NR. X265
EXP. NR. X290

Fig. 2

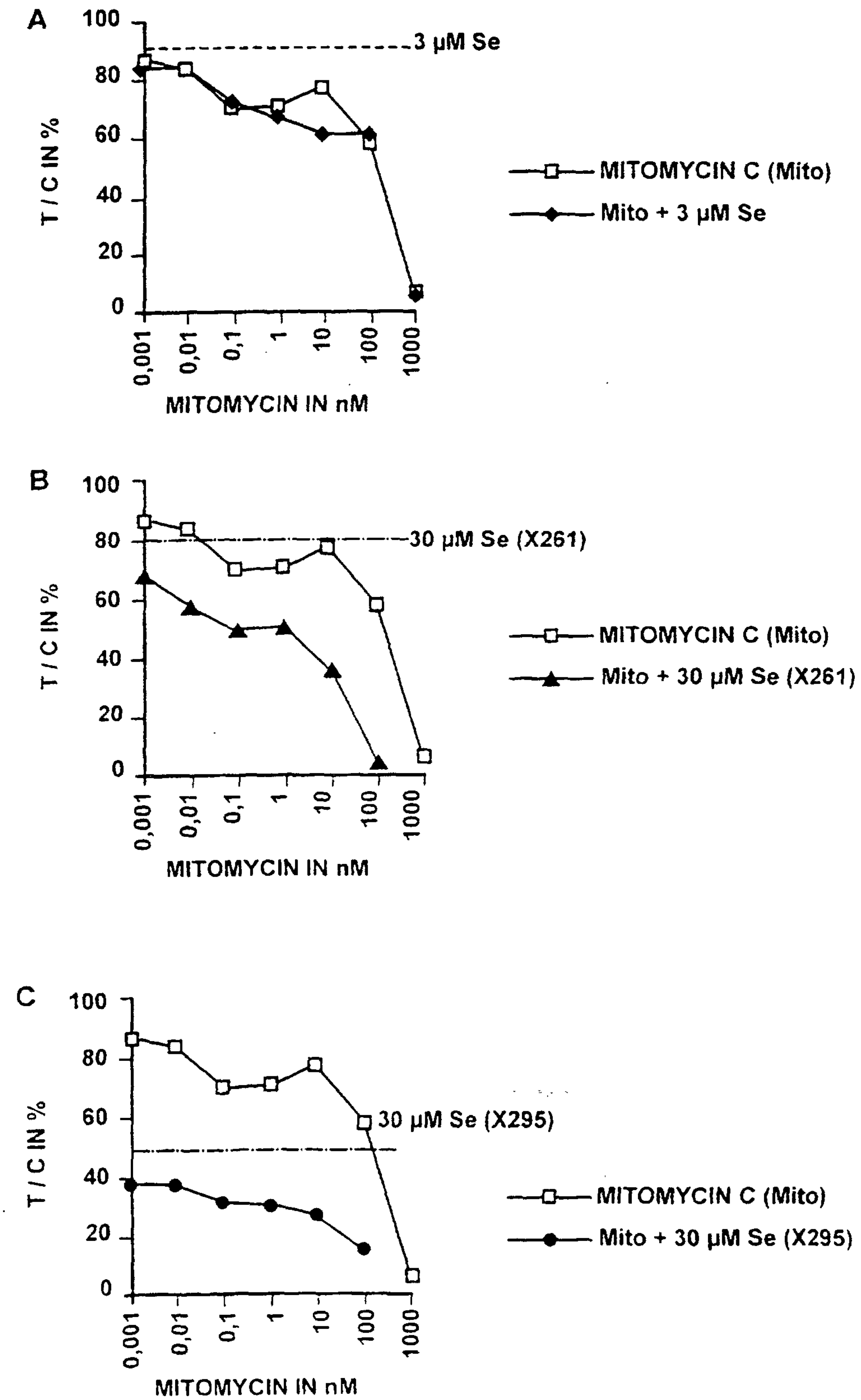
A
100
80
60
40
20
0
T / C IN %
3 µM Se
0,001
0,01
0,1
1
10
100
1000
MITOMYCIN IN nM
MITOMYCIN C (Mito)
Mito + 3 µM Se
B
30 µM Se (X261)
MITOMYCIN C (Mito)
Mito + 30 µM Se (X261)
C
30 µM Se (X295)
MITOMYCIN C (Mito)
Mito + 30 µM Se (X295)

*Fig. 3*

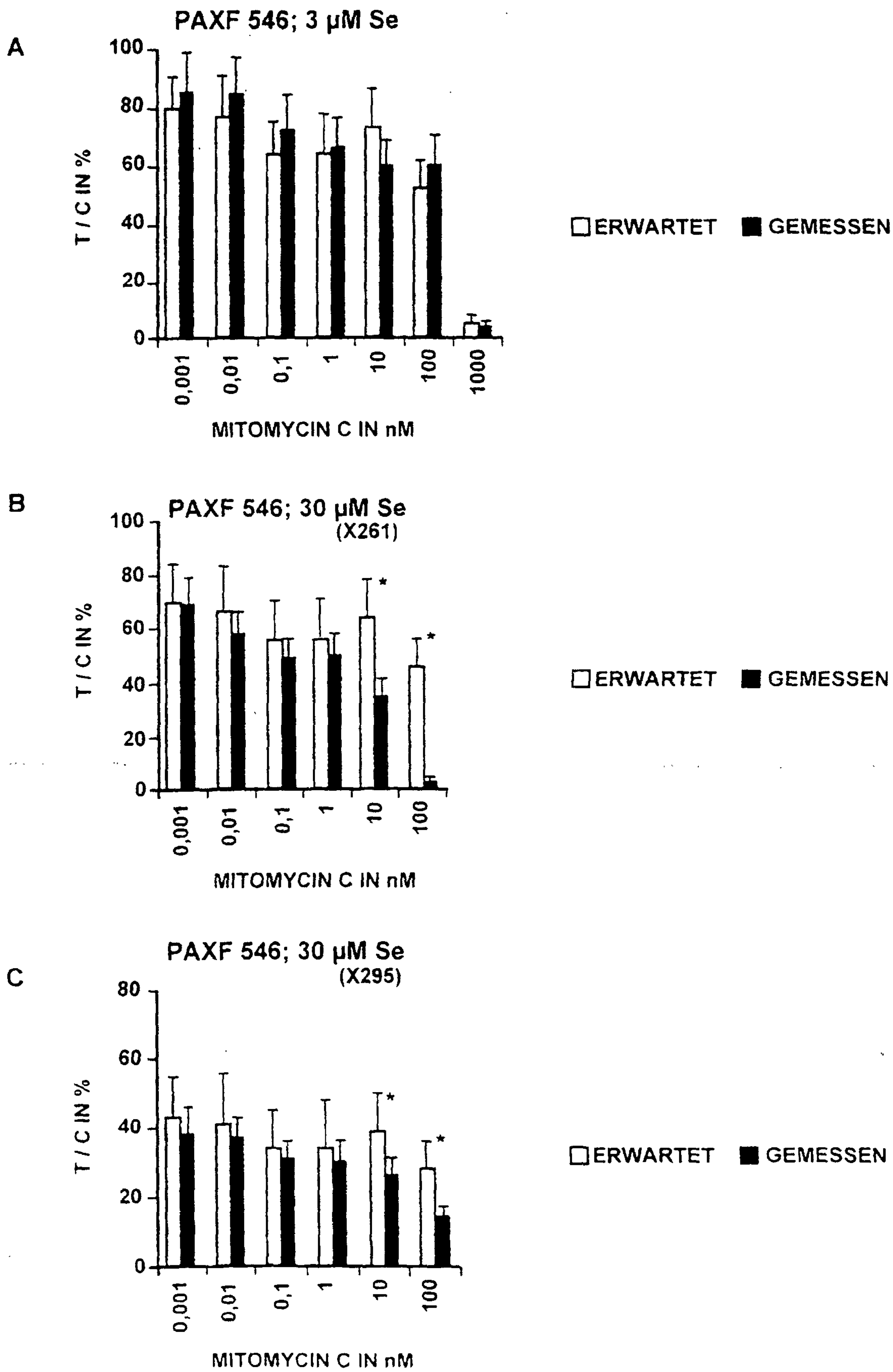
A
PAXF 546; 3 µM Se
T / C IN %
100
80
60
40
20
0
0,001
0,01
0,1
1
10
100
1000
MITOMYCIN C IN nM
ERWARTET
GEMESSEN
B
PAXF 546; 30 µM Se
(X261)
T / C IN %
100
80
60
40
20
0
*
*
0,001
0,01
0,1
1
10
100
MITOMYCIN C IN nM
ERWARTET
GEMESSEN
C
PAXF 546; 30 µM Se
(X295)
T / C IN %
80
60
40
20
0
*
*
0,001
0,01
0,1
1
10
100
MITOMYCIN C IN nM
ERWARTET
GEMESSEN

*Fig. 4*

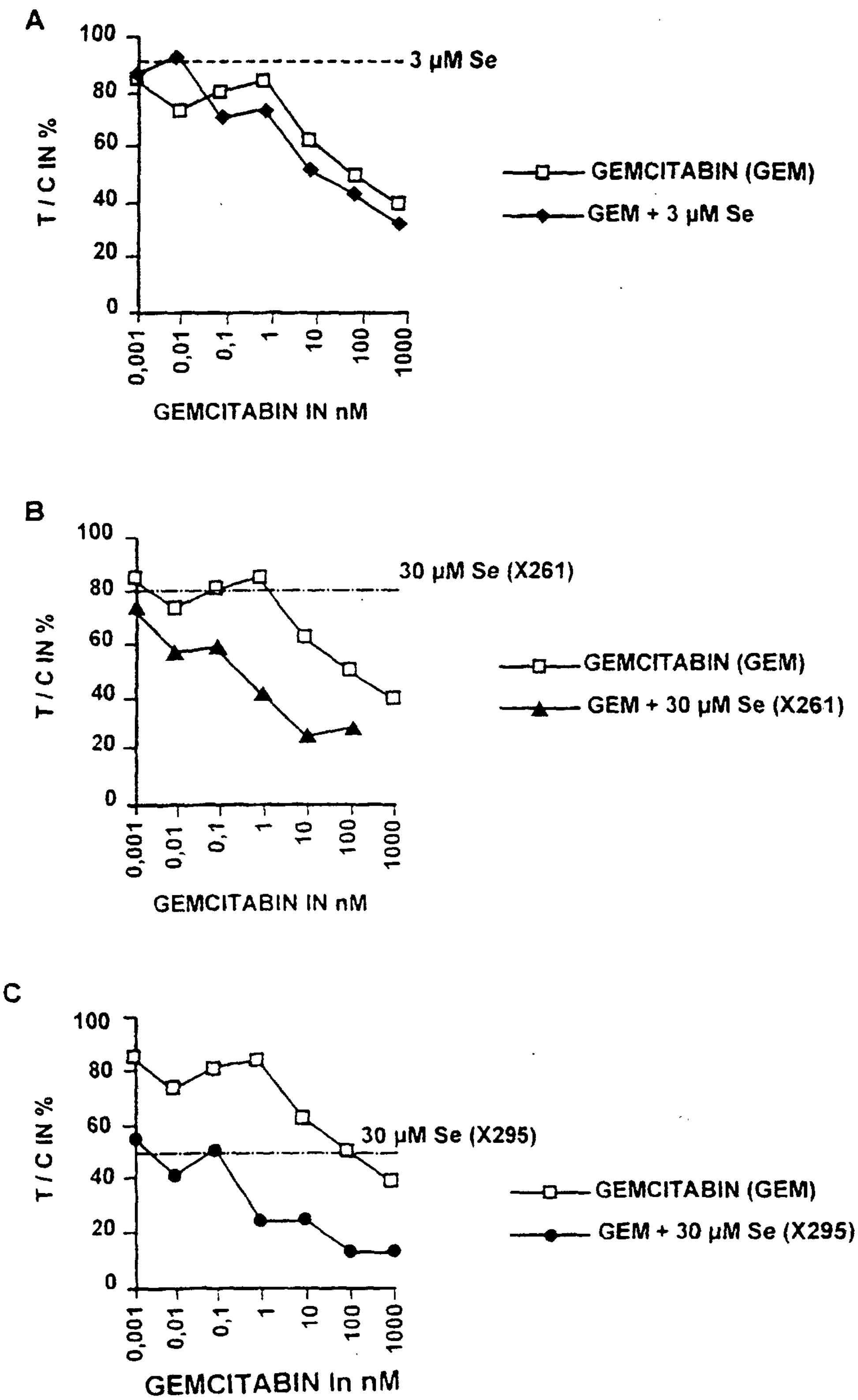
A
100
80
60
40
20
0
T / C IN %
3 µM Se
GEMCITABIN (GEM)
GEM + 3 µM Se
0,001
0,01
0,1
1
10
100
1000
GEMCITABIN IN nM
B
100
80
60
40
20
0
T / C IN %
30 µM Se (X261)
GEMCITABIN (GEM)
GEM + 30 µM Se (X261)
0,001
0,01
0,1
1
10
100
1000
GEMCITABIN IN nM
C
100
80
60
40
20
0
T / C IN %
30 µM Se (X295)
GEMCITABIN (GEM)
GEM + 30 µM Se (X295)
0,001
0,01
0,1
1
10
100
1000
GEMCITABIN In nM

Fig. 5

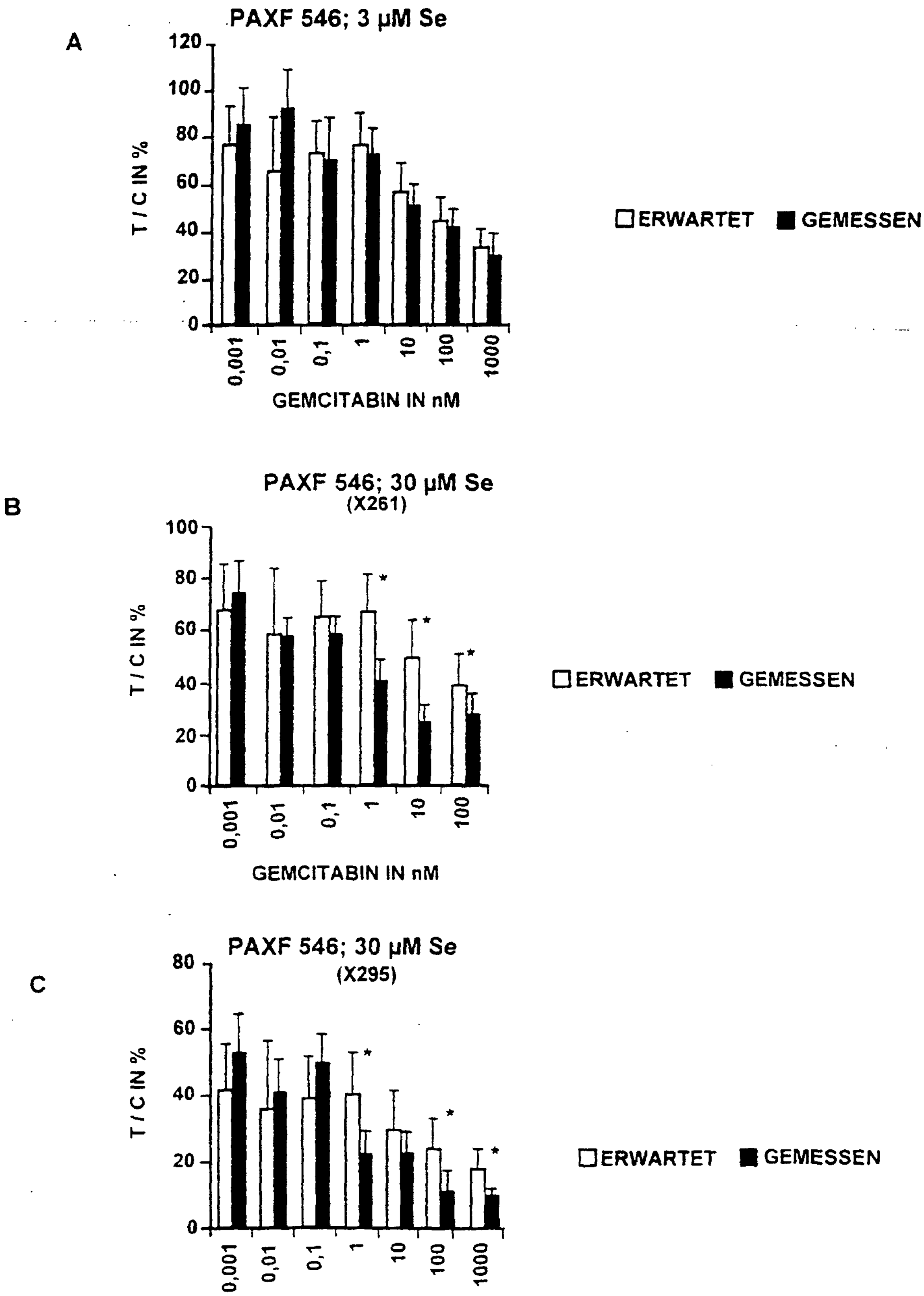
A
PAXF 546; 3 µM Se
T / C IN %
GEMCITABIN IN nM
ERWARTET
GEMESSEN
B
PAXF 546; 30 µM Se
(X261)
T / C IN %
GEMCITABIN IN nM
ERWARTET
GEMESSEN
C
PAXF 546; 30 µM Se
(X295)
T / C IN %
ERWARTET
GEMESSEN